# EUROPEAN PATENT APPLICATION

(11) **EP 3 473 719 A1**
(43) Date of publication of application: **24.04.2019**
(21) Application number: 17812440.0
(22) Date of filing: 07.04.2017
(51) Int. Cl.: C12N 15/11, C12N 5/10, C12Q 1/68, A01H 5/00, A01H 1/02, A01N 57/20, A01N 25/32, A01P 13/00

(54) **NUCLEIC ACID SEQUENCE FOR DETECTING EXISTENCE OF TRANSGENIC SOYBEAN EVENT DBN9004 IN BIOLOGICAL SAMPLE, KIT CONTAINING SAME AND DETECTION METHOD THEREFOR**

(30) Priority: 18.06.2016 CN 201610440310
(71) Applicant: Beijing Dabeinong Technology Group Co., Ltd., Beijing 100080 (CN); Beijing Dabeinong Biotechnology Co.,Ltd, Beijing 100193 (CN)
(72) Inventor: WANG, Dengyuan, Beijing 100193 (CN); YU, Caihong, Beijing 100193 (CN); ZHANG, Chengwei, Beijing 100193 (CN); HAN, Chao, Beijing 100193 (CN); LI, Xiaojiao, Beijing 100193 (CN); JIANG, Ziqin, Beijing 100193 (CN); ZHANG, Liangjun, Beijing 100193 (CN); WU, Zhujun, Beijing 100193 (CN); TIAN, Kangle, Beijing 100193 (CN); BAO, Xiaoming, Beijing 100193 (CN)
(74) Representative: Studio Torta S.p.A.
(86) International application number: PCT/CN2017/079658
(87) International publication number: WO 2017/215328

(57) **Abstract**

Provided are a nucleic acid sequence for detecting the existence of a transgenic soybean event DBN9004 in a biological sample, a kit comprising the same and a detection method thereof.

## Description

### Technical Field

The present invention relates to a nucleic acid sequence for the detection of a herbicide tolerant soybean plant DBN9004 and a detection method thereof, in particular to a soybean plant DBN9004 tolerant to glyphosate and glufosinate and a method for detecting whether a DNA molecule of the particular transgenic soybean event DBN9004 is contained in a biological sample.

### Background

N-phosphonomethylglycine, also known as glyphosate, is a systemically translocated chronic broad-spectrum non-selective herbicide. Glyphosate is a competitive inhibitor of phosphoenolpyruvic acid (PEP), a substrate of 5-enolpyruvylshikimate-3-phosphate synthase (EPSPS) in synthesis, and can inhibit the conversion of the two substrates PEP and 3-phosphoshikimate to 5-enolpyruvylshikimate-3-phosphoshikimate catalyzed by EPSPS, thereby blocking the synthetic pathway of shikimic acid, an aromatic amino acid synthesis precursor, and causing the death of plants and bacteria due to the interference of the synthesis of proteins.

Glyphosate tolerance can be achieved by expressing the modified *EPSPS.* The modified *EPSPS* has a lower affinity to glyphosate, and therefore in the presence of glyphosate, *EPSPS* maintains its catalytic activity, i.e., obtaining glyphosate tolerance.

Soybean (*Glycine max*) is one of the world's five main crops. Herbicide tolerance, particularly the tolerance to a glyphosate herbicide, is an important agronomic trait in soybean production. Soybean tolerance to a glyphosate herbicide can be obtained by transgenic methods for expressing glyphosate herbicide-tolerant genes (*EPSPS, CP4*) in soybean plants such as soybean event GTS40-3-2, and soybean event MON89788.

In recent years, the widespread adoption of glyphosate tolerant farming systems and the increasing use of glyphosate have led to the prevalence of glyphosate-resistant weeds. In areas where planters face glyphosate-resistant weeds or weeds which transform to more difficult-to-control weed species, planters can compensate for the weakness of glyphosate by mixing or alternating with other herbicides capable of controlling the missing weeds.

Glufosinate is a non-systematic and non-selective herbicide in phosphinothricin herbicides. It is mainly used for the post-emergence control of annual or perennial broadleaf weeds, and the control of weeds is realized through the irreversible inhibition of L-phosphonomycin (an active ingredient in glufosinate) on glutamine synthase (an enzyme essential for ammonia detoxification in plants). Different from glyphosate which can kill the roots of plants, glufosinate can kill leaves first and translocate in the xylem of plants by transpiration, and its fast-acting property is between paraquat and glyphosate.

L-phosphinothricin is converted to its inactive form through acetylation catalyzed by phosphinothricin N-acetyltransferase (PAT) isolated from Streptomyces. Genes expressing plant optimized forms of PAT have been used in soybeans to impart tolerance to a glufosinate herbicide to soybeans such as soybean event A5547-127. Thus, the use of glufosinate herbicides in combination with glufosinate-tolerant traits can serve as a non-selective means for the effective management of glyphosate-resistant weeds.

In the future, with the popularization of transgenic insect-resistant soybeans and their large-scale planting, a small number of insects/pests survived may produce resistance after several generations of propagation. Co-planting transgenic herbicide tolerant soybeans as non insect-resistant transgenic soybeans with transgenic insect-resistant soybeans in a proportion can delay the induction of insect/pest resistance.

It is known that the expression of exogenous genes in plants is affected by their chromosomal location, which is possibly due to the proximity of the chromatin structures (e.g., heterochromatin) or transcriptional regulatory elements (e.g., enhancers) to the integration site. To this end, it is often necessary to screen a large number of events before it is possible to identify events that can be commercialized (i.e., events where the target genes introduced can be optimally expressed). For example, it has been observed in plants and other organisms that there may be great difference in the expression quantities of the introduced genes between events; there may also be difference in spatial or temporal patterns of expression, such as difference in the relative expression of transgenes among different plant tissues, such difference being manifested by the fact that the actual expression pattern may be inconsistent with the expression pattern expected according to the transcriptional regulatory elements in the introduced gene constructs. Thus, it is generally necessary to generate hundreds of different events and to screen out a single event with transgene expression quantities and expression patterns that are intended for commercialization from these events. Events with expected transgene expression quantities and expression patterns can be used to introduce transgenes into other genetic backgrounds by means of sexual heterotypic hybridization using conventional breeding methods. The progenies produced by this hybridization approach retain the transgene expression characteristics of the original transformants. Application of this strategy pattern can ensure reliable gene expression in many varieties, and these varieties can be well adapted to the local growth conditions.

It will be beneficial that the presence of a particular event can be detected to determine whether the progenies of the sexual hybridization contain the target genes. In addition, methods for detecting specific events will also help to comply with relevant regulations, for example, the formal approval and labelling of foods derived from recombinant crops would be required before they are put on the market. It is possible to detect the presence of transgenes by any well-known polynucleotide detection method, for example, polymerase chain reactions (PCRs) or DNA hybridization using a polynucleotide probe. These detection methods generally focus on common genetic elements such as promoters, terminators, and marker genes. Thus, unless the sequence of chromosomal DNA ("flanking DNA") adjacent to the inserted transgenic DNA is known, such methods above would not be able to differentiate different events, particularly those generated with the same DNA construct. Therefore, a pair of primers that span the conjugation site of the inserted transgene and the flanking DNA, in particular, a first primer containing the flanking sequence and a second primer containing the inserted sequence, are often used to identify transgenic specific events by PCR at present.

### Summary of the Invention

The objective of the present invention is to provide a nucleic acid sequence for the detection of a herbicide tolerant soybean plant DBN9004 and a detection method thereof, the transgenic soybean event DBN9004 has better tolerance to a glyphosate herbicide and a glufosinate herbicide, and the detection method can accurately and quickly identify whether a DNA molecule of the specific transgenic soybean event DBN9004 is contained in a biological sample.

In order to achieve the above objective, the present invention provides a nucleic acid molecule having the following nucleic acid sequence, the nucleic acid sequence comprising at least 11 consecutive nucleotides in SEQ ID NO: 3 or its complementary sequence, and/or at least 11 consecutive nucleotides in SEQ ID NO: 4 or its complementary sequence.

Preferably, the nucleic acid sequence comprises SEQ ID NO: 1 or its complementary sequence, and/or SEQ ID NO: 2 or its complementary sequence.

Further, the nucleic acid sequence comprises SEQ ID NO: 3 or its complementary sequence, and/or SEQ ID NO: 4 or its complementary sequence.

Still further, the nucleic acid sequence comprises SEQ ID NO: 5 or its complementary sequence.

The SEQ ID NO: 1 or its complementary sequence is a sequence with a length of 22 nucleotides located at the 5' terminus of the inserted sequence near the insertion conjugation site in the transgenic soybean event DBN9004, and the SEQ ID NO: 1 or its complementary sequence spans the flanking genomic DNA sequence of the soybean insertion site and the DNA sequence at the 5' terminus of the inserted sequence; thus the inclusion of the SEQ ID NO: 1 or its complementary sequence can be identified as the presence of transgenic soybean event DBN9004. The SEQ ID NO: 2 or its complementary sequence is a sequence with a length of 22 nucleotides located at the 3' terminus of the inserted sequence near the insertion conjugation site in the transgenic soybean event DBN9004, and the SEQ ID NO: 2 or its complementary sequence spans the DNA sequence at the 3' terminus of the inserted sequence and the flanking genomic DNA sequence of the soybean insertion site; thus the inclusion of the SEQ ID NO: 2 or its complementary sequence can be identified as the presence of transgenic soybean event DBN9004.

In the present invention, the nucleic acid sequence can be at least 11 or more consecutive polynucleotides (a first nucleic acid sequence) of any part of the transgenic inserted sequence in the SEQ ID NO: 3 or its complementary sequence, or at least 11 or more consecutive polynucleotides (a second nucleic acid sequence) of any part of the 5' flanking soybean genomic DNA region in the SEQ ID NO: 3 or its complementary sequence. The nucleic acid sequence can further be a part homologous to or complementary to the SEQ ID NO: 3 comprising the intact SEQ ID NO: 1. When the first nucleic acid sequence and the second nucleic acid sequence are used together, these nucleic acid sequences as a DNA primer pair can be used in the DNA amplification method for producing an amplification product. The presence of the transgenic soybean event DBN9004 or its progenies can be diagnosed when the amplification product produced in the DNA amplification method using the DNA primer pair is an amplification product comprising SEQ ID NO: 1. It is well known to a person skilled in the art that the first and second nucleic acid sequences are not necessary to be consisted solely of DNA, but can also comprise RNA, a mixture of DNA and RNA, or a combination of DNA, RNA, or other nucleotides or analogues thereof which are not as templates of one or more polymerases. Furthermore, the probes or primers described in the present invention should be at least about 11, 12, 13, 14, 15, 16, 17, 18, 19, 20, 21 or 22 consecutive nucleotides in length, which can be selected from nucleotides shown as SEQ ID NO: 1, SEQ ID NO: 2, SEQ ID NO: 3, SEQ ID NO: 4 and SEQ ID NO: 5. When selected from the nucleotides shown as SEQ ID NO: 3, SEQ ID NO: 4, and SEQ ID NO: 5, the probes and primers may be consecutive nucleotides having a length of at least about 21 to about 50 or more. The SEQ ID NO: 3 or its complementary sequence is a sequence with a length of 1207 nucleotides located at the 5' terminus of the inserted sequence near the insertion conjugation site in the transgenic soybean event DBN9004, and the SEQ ID NO: 3 or its complementary sequence consists of the soybean flanking genomic DNA sequence of 740 nucleotides (nucleotides 1-740 of SEQ ID NO: 3), 74 nucleotides in the DNA sequence of the pDBN4003 construct (nucleotides 741-814 of SEQ ID NO: 3), t35S terminator sequence of 195 nucleotides (nucleotides 815-1009 of SEQ ID NO: 3), a vector spacer sequence of 21 nucleotides (nucleotides 1010-1030 of SEQ ID NO: 3), and the 5' terminal DNA sequence of phosphinothricin N-acetyltransferase cPAT of 177 nucleotides (nucleotides 1031-1207 of SEQ ID NO: 3) (nucleotides 815-1207 of SEQ ID NO: 3); thus the inclusion of the SEQ ID NO: 3 or its complementary sequence can be identified as the presence of transgenic soybean event DBN9004.

The nucleic acid sequence can be at least 11 or more consecutive polynucleotides (a third nucleic acid sequence) of any part of the transgenic inserted sequence in the SEQ ID NO: 4 or its complementary sequence, or at least 11 or more consecutive polynucleotides (a fourth nucleic acid sequence) of any part of the 3' flanking soybean genomic DNA region in the SEQ ID NO: 4 or its complementary sequence. The nucleic acid sequence can further be a part homologous to or complementary to the SEQ ID NO: 4 comprising the intact SEQ ID NO: 2. When the third nucleic acid sequence and the fourth nucleic acid sequence are used together, these nucleic acid sequences as a DNA primer pair can be used in the DNA amplification method for producing an amplification product. The presence of the transgenic soybean event DBN9004 or its progenies can be diagnosed when the amplification product produced in the DNA amplification method using the DNA primer pair is an amplification product comprising SEQ ID NO: 2. The SEQ ID NO: 4 or its complementary sequence is a sequence with a length of 631 nucleotides located at the 3' terminus of the inserted sequence near the insertion conjugation site in the transgenic soybean event DBN9004, and the SEQ ID NO: 4 or its complementary sequence consists of the prGm17gTsf1 promoter sequence of 226 nucleotides (nucleotides 1-226 of SEQ ID NO: 4), 61 nucleotides in the DNA sequence of the pDBN4003 construct (nucleotides 227-287 of SEQ ID NO: 4), a conjugating sequence of 6 nucleotides (nucleotides 288-293 of SEQ ID NO: 4) and the soybean flanking genomic DNA sequence of 338 nucleotides (nucleotides 294-631 of SEQ ID NO: 4); thus the inclusion of the SEQ ID NO: 4 or its complementary sequence can be identified as the presence of transgenic soybean event DBN9004.

The SEQ ID NO: 5 or its complementary sequence is a sequence of 7059 nucleotides in length which characterizes the transgenic soybean event DBN9004, the genomes and genetic elements specifically contained in which are as shown in Table 1. The inclusion of the SEQ ID NO: 5 or its complementary sequence can be identified as the presence of transgenic soybean event DBN9004.

**Table 1. Genomes and genetic elements contained in SEQ ID NO: 5**

| Genetic element/genome | Length (bp) | Position located on SEQ ID NO: 5 |
|---|---|---|
| 5' genome | 1268 | 1-1268 |
| LB | 74 | 1269-1342 |
| t35S | 195 | 1343-1537 |
| cPAT | 552 | 1559-2110 |
| pr35s | 530 | 2130-2659 |
| tPse9 | 643 | 2708-3350 |
| cEPSPS | 1368 | 3393-4760 |
| spAt CTP2 | 228 | 4761-4988 |
| prGm17gTsf1 | 1012 | 4989-6000 |
| RB | 61 | 6001-6061 |
| 3' genome | 992 | 6068-7059 |

The nucleic acid sequences or their complementary sequences can be used in the DNA amplification method to produce amplicons, the detection of the amplicons diagnoses the presence of the transgenic soybean event DBN9004 or progenies thereof in a biological sample; and the nucleic acid sequences or their complementary sequences can be used in the nucleotide detecting methods to detect the presence of the transgenic soybean event DBN9004 or progenies thereof in a biological sample.

In order to achieve the above objective, the present invention also provides a method for detecting the presence of the DNA of transgenic soybean event DBN9004 in a sample, comprising:
contacting a sample to be detected with at least two types of primers for amplifying a target amplification product in a nucleic acid amplification reaction;
performing a nucleic acid amplification reaction; and
detecting the presence of the target amplification product;
the target amplification product comprises at least 11 consecutive nucleotides in SEQ ID NO: 3 or its complementary sequence, and/or at least 11 consecutive nucleotides in SEQ ID NO: 4 or its complementary sequence.

Further, the target amplification product comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

Still further, the target amplification product comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence.

In the above-mentioned technical solutions, at least one of the primers comprises the nucleic acid sequence or a fragment thereof, or a sequence complementary thereto.

Specifically, the primers comprise a first primer and a second primer, wherein the first primer is selected from SEQ ID NO: 8 and SEQ ID NO: 10; and the second primer is selected from SEQ ID NO: 9 and SEQ ID NO: 11.

In order to achieve the above objective, the present invention also provides a method for detecting the presence of the DNA of transgenic soybean event DBN9004 in a sample, comprising:
contacting a sample to be detected with a probe, the probe comprising at least 11 consecutive nucleotides in SEQ ID NO: 3 or its complementary sequence, and/or at least 11 consecutive nucleotides in SEQ ID NO: 4 or its complementary sequence;
hybridizing the sample to be detected with the probe under stringent hybridization conditions; and
detecting the hybridization status between the sample to be detected and the probe.

The stringent conditions may be performing the hybridization in a solution of 6 × SSC (sodium citrate) and 0.5% of SDS (lauryl sodium sulfate) at 65°C, then washing the membrane once with 2 × SSC, 0.1% of SDS, and 1 × SSC, 0.1% SDS, respectively.

Further, the probe comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

Still further, the probe comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence. Optionally, at least one of the probes is labelled with at least one fluorophore.

In order to achieve the above objective, the present invention also provides a method for detecting the presence of the DNA of transgenic soybean event DBN9004 in a sample, comprising:
contacting the sample to be detected with a marker nucleic acid molecule, the marker nucleic acid molecule comprising at least 11 consecutive nucleotides in SEQ ID NO: 3 or its complementary sequence, and/or at least 11 consecutive nucleotides in SEQ ID NO: 4 or its complementary sequence;
hybridizing the sample to be detected with the marker nucleic acid molecule under stringent hybridization conditions; and
detecting the hybridization status of the sample to be detected with the marker nucleic acid molecule, and further determining the gene linkage between the glyphosate tolerance and/or glufosinate tolerance and the marker nucleic acid molecule in genetics by the marker-assisted breeding analysis.

Further, the marker nucleic acid molecule comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

Still further, the marker nucleic acid molecule comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence.

In order to achieve the above objective, the present invention also provides a DNA detection kit, comprising at least one DNA molecule, wherein the DNA molecule comprises at least 11 consecutive nucleotides of a homologous sequence of SEQ ID NO: 3 or its complementary sequence, and/or at least 11 consecutive nucleotides of a homologous sequence of SEQ ID NO: 4 or its complementary sequence, and can be served as a DNA primer or a probe specific for the transgenic soybean event DBN9004 or its progenies.

Further, the DNA molecule comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

Still further, the DNA molecule comprises at least one selected from: a homologous sequence of SEQ ID NO: 1 or its complementary sequence, a homologous sequence of SEQ ID NO: 2 or its complementary sequence, a homologous sequence of SEQ ID NO: 6 or its complementary sequence, and a homologous sequence of SEQ ID NO: 7 or its complementary sequence.

In order to achieve the above objective, the present invention also provides a plant cell or part, comprising a nucleic acid sequence encoding a glyphosate-tolerant EPSPS protein, a nucleic acid sequence encoding a glufosinate-tolerant PAT protein, and a nucleic acid sequence of a specific region. The nucleic acid sequence of the specific region comprises at least one selected from: the sequences as shown in SEQ ID NO: 1, SEQ ID NO: 2, SEQ ID NO: 6, and SEQ ID NO: 7.

In order to achieve the above objective, the present invention also provides a method for producing a soybean plant tolerant to a glyphosate herbicide and/or a glufosinate herbicide, comprising introducing into the genome of the soybean plant a nucleic acid sequence encoding a glyphosate-tolerant EPSPS protein and/or a nucleic acid sequence encoding a glufosinate-tolerant PAT protein, and a nucleic acid sequence of a specific region selected from at least one nucleic acid sequence of the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7.

Specifically, the method for producing a soybean plant tolerant to a glyphosate herbicide and/or a glufosinate herbicide comprises:
sexually hybridizing a first parent soybean plant of the transgenic soybean event DBN9004 which is tolerant to a glyphosate herbicide and/or a glufosinate herbicide with a second parent soybean plant which lacks glyphosate and/or glufosinate tolerance to produce a large number of progeny plants;
treating the progeny plants with a glyphosate herbicide and/or a glufosinate herbicide; and
selecting the progeny plants which are tolerant to glyphosate and/or glufosinate.

In order to achieve the above objective, the present invention also provides a method for cultivating a soybean plant tolerant to a glyphosate herbicide and/or a glufosinate herbicide, comprising:
planting at least one soybean seed, the genome of the soybean seed comprising a nucleic acid sequence encoding a glyphosate-tolerant EPSPS protein and/or a nucleic acid sequence encoding a glufosinate-tolerant PAT protein, and a nucleic acid sequence of a specific region;
growing the soybean seed into a soybean plant; and
spraying the soybean plant with an effective dose of a glyphosate herbicide and/or a glufosinate herbicide and harvesting the plant with reduced plant damage compared to other plants without the nucleic acid sequence of the specific region;
the nucleic acid sequence of the specific region is selected from at least one nucleic acid sequence of the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7.

In order to achieve the above objective, the present invention also provides a method for protecting a plant from damage caused by a herbicide, comprising applying a herbicide containing an effective dose of glyphosate and/or glufosinate to a field for planting at least one transgenic soybean plant, wherein the transgenic soybean plant comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome, and the transgenic soybean plant has tolerance to the glyphosate herbicide and/or the glufosinate herbicide.

In order to achieve the above objective, the present invention also provides a method for controlling field weeds, comprising applying a herbicide containing an effective dose of glyphosate and/or glufosinate to a field for planting at least one transgenic soybean plant, wherein the transgenic soybean plant comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome, and the transgenic soybean plant has tolerance to the glyphosate herbicide and/or the glufosinate herbicide.

In order to achieve the above objective, the present invention also provides a method for controlling glyphosate resistant weeds in a field for a glyphosate tolerant plant, comprising applying a herbicide containing an effective dose of glufosinate to a field for planting at least one glyphosate tolerant transgenic soybean plant, wherein the glyphosate tolerant transgenic soybean plant comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome, and the glyphosate tolerant transgenic soybean plant has tolerance to the glufosinate herbicide at the same time.

In order to achieve the above objective, the present invention also provides a method for delaying insect resistance, comprising growing at least one transgenic soybean plant having glyphosate and/or glufosinate tolerance in a field for planting an insect resistant soybean plant, wherein the transgenic soybean plant having glyphosate and/or glufosinate tolerance comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome.

In order to achieve the above objective, the present invention also provides an agricultural product or commodity comprising a polynucleotide of SEQ ID NO: 1 or SEQ ID NO: 2, the agricultural product or commodity is lecithin, a fatty acid, glycerol, a sterol, a soybean flake, soy flour, a soy protein or its concentrate, a soybean oil, a soy protein fiber, a soy milk clot or bean curd.

In the nucleic acid sequence for detecting the herbicide-tolerant soybean plant DBN9004 and a detection method thereof in the present invention, the following definitions and methods can better define the present invention and guide a person skilled in the art to practice the present invention, and unless otherwise stated, the terms herein are understood according to conventional usages by an ordinary person skilled in the art.

The term "soybean" refers to *Glycine max* and comprises all plant species that can mate with the soybean, including wild soybean species.

The term "comprise" or "include" refers to "including but not limited to".

The term "plant" includes a whole plant, a plant cell, a plant organ, a plant protoplast, a plant cell tissue culture from which a plant can be regenerated, a plant callus, a plant clump and an intact plant cell in a plant or plant part such as an embryo, a pollen, an ovule, a seed, a leaf, a flower, a branch, a fruit, a stalk, a root, a root tip, and an anther. It should be understood that a part of a transgenic plant within the scope of the present invention includes but is not limited to, a plant cell, a protoplast, a tissue, a callus, an embryo and a flower, a stem, a fruit, a leaf and a root, and the above plant parts are derived from the transgenic plants that are transformed in advance with the DNA molecule of the present invention and thus at least partially composed of the transgenic cells, or their progenies.

The term "gene" refers to a nucleic acid fragment that expresses a particular protein, including a regulatory sequence (5' non-coding sequence) prior to the coding sequence and a regulatory sequence (3' non-coding sequence) after the coding sequence. The term "natural gene" refers to a gene that is naturally found to have its own regulatory sequences. The term "chimeric gene" refers to any gene that is not a natural gene, which contains non-naturally occurring regulatory and coding sequences. The term "endogenous gene" refers to a natural gene that is located in its natural position in the genome of an organism. The term "exogenous gene" is a foreign gene that is now present in the genome of an organism but is not originally present, and also refers to a gene that is introduced into a receptor cell through transgenic steps. The exogenous gene may comprise a natural gene or a chimeric gene inserted into a non-natural organism. The term "transgene" is a gene that has been introduced into a genome through a transformation procedure. The site at which the recombinant DNA has been inserted in the plant genome may be referred to as an "insertion site" or "target site".

The term "flanking DNA" may comprise a genome that naturally exists in, for example, a plant organism or an exogenous (heterologous) DNA introduced through a transformation process, such as a fragment associated with a transformation event. Thus, the flanking DNA can include a combination of natural and exogenous DNAs. In the present invention, the "flanking region" or "flanking sequence" or "genomic boundary region" or "genomic boundary sequence" refers to a sequence of at least 3, 5, 10, 11, 15, 20, 50, 100, 200, 300, 400, 1000, 1500, 2000, 2500, or 5000 base pairs or a longer sequence, which locates at the direct upstream or downstream of the initially exogenously inserted DNA molecule and adjacent to the initially exogenously inserted DNA molecule. When the flanking region locates at the downstream, it may also be referred to as "left boundary flank" or "3' flank" or "3' genome boundary region" or "genome 3' boundary sequence", and the like. When the flanking region locates at the upstream, it may also be referred to as "right boundary flank" or "5' flank" or "5' genome boundary region" or "genome 5' boundary sequence", and the like.

The transformation procedure causing a random integration of exogenous DNA will result in transformants containing different flanking regions that are specifically contained in each transformant. When the recombinant DNA is introduced into a plant by conventional hybridization, its flanking region usually will not be changed. The transformants will also contain unique conjugation between heterogeneous insert DNA and a segment of genomic DNA or between two segments of genomic DNAs or between two segments of heterogeneous DNAs. "Conjugation" is the point at which two specific DNA fragments are linked. For example, the conjugation exists in the position where the insert DNA is linked to the flanking DNA. The conjugation points are also present in the transformed organisms, in which the two DNA fragments are linked together in a manner that is found in modified natural organisms. "Conjugating DNA" or "conjugating region" refers to a DNA containing a conjugation point.

The present invention provides a transgenic soybean event called DBN9004 and progenies thereof, the transgenic soybean event DBN9004 is a soybean plant DBN9004 which comprises plants and seeds of the transgenic soybean event DBN9004 and plant cells or regenerable parts thereof. The plant parts of the transgenic soybean event DBN9004 include but are not limited to cells, pollens, ovules, flowers, buds, roots, stems, leaves, pods and products derived from the soybean plant DBN9004 such as soybean cakes, powders and oils, specifically lecithin, fatty acids, glycerol, sterols, edible oils, defatted soy flakes, defatted and baked soy flours, soy milk clots, bean curd, soy protein concentrates, isolated soy proteins, hydrolyzed vegetable proteins, organized soy proteins and soy protein fibers.

The transgenic soybean event DBN9004 of the present invention comprises a DNA construct, the transgenic soybean event DBN9004 acquires the tolerance to a glyphosate herbicide and a glufosinate herbicide when the DNA construct is expressed in plant cells. The DNA construct comprises two tandem expression cassettes, wherein the first expression cassette comprises a suitable promoter and a suitable polyadenylation signal sequence for expression in a plant, the promoter can be operably linked to a gene encoding 5-enolpyruvylshikimate-3-phosphate synthase (EPSPS), and the *EPSPS* is tolerant to a glyphosate herbicide. The second expression cassette comprises a suitable promoter and a suitable polyadenylation signal sequence for expression in a plant, the promoter can be operably linked to a gene encoding phosphinothricin N-acetyltransferase (PAT), and the nucleic acid sequence of the PAT protein is tolerant to a glufosinate herbicide. Further, the promoter may be a suitable promoter isolated from a plant, including constitutive, inducible and/or tissue-specific promoters, and the suitable promoter includes but is not limited to, cauliflower mosaic virus (CaMV) 35S promoter, figwort mosaic virus (FMV) 35S promoter, Tsfl promoter, ubiquitin promoter, actin promoter, *Agrobacterium tumefaciens* nopaline synthase (NOS) promoter, octopine synthase (OCS) promoter, Cestrum yellow leaf curl virus promoter, Patatin promoter, ribulose-1,5-bisphosphate carboxylase/oxygenase (RuBisCO) promoter, glutathione S-transferase (GST) promoter, E9 promoter, GOS promoter, alcA/alcR promoter, *Agrobacterium rhizogenes* RolD promoter and Arabidopsis Suc2 promoter. The polyadenylation signal sequence may be a suitable polyadenylation signal sequence that functions in plants, and the suitable polyadenylation signal sequence includes but is not limited to, the polyadenylation signal sequence derived from the *Agrobacterium tumefaciens* nopaline synthase (NOS) gene, the polyadenylation signal sequences derived from the cauliflower mosaic virus (CaMV) 35S terminator and derived from the pea ribulose-1,5-bisphosphate carboxylase/oxygenase E9 terminator, the polyadenylation signal sequence derived from the protease inhibitor II (PINII) gene, and the polyadenylation signal sequence derived from the α-tubulin gene.

In addition, the expression cassette may also include other genetic elements including but are not limited to, enhancers and signal peptides/transit peptides. The enhancer may enhance the expression level of the gene, and the enhancer includes but is not limited to, the tobacco etch virus (TEV) translation activating factor, CaMV35S enhancer, and FMV35S enhancer. The signal peptide/transit peptide may direct the EPSPS protein and/or the PAT protein to be transported to an extracellular or intracellular specific organelle or compartment, for example, the chloroplast is targeted using a sequence encoding a chloroplast transit peptide, or the endoplasmic reticulum is targeted using 'KDEL' retention sequence.

The 5-enolpyruvylshikimate-3-phosphate synthase (EPSPS) gene may be isolated from the *Agrobacterium tumefaciens* CP4 strain, and the polynucleotides encoding EPSPS may be altered by optimizing the codons or through other manners to achieve the purpose of increasing the stability and availability of transcripts in the transformed cells. The 5-enolpyruvylshikimate-3-phosphate synthase (EPSPS) gene may also be used as a selectable maker gene.

The "glyphosate" refers to N-phosphonomethylglycine and salts thereof. Treating with a "glyphosate herbicide" refers to performing treatment using any glyphosate-containing herbicide preparation. The choice of usage rate of a certain glyphosate preparation in order to achieve an effective biological dose will not exceed the skills of an ordinary agronomic technician. Treating a field including plant materials derived from the herbicide-tolerant soybean plant DBN9004 using any glyphosate-containing herbicide preparation will control the growth of weeds in the field and will not affect the growth or yield of the plant materials derived from the herbicide-tolerant soybean plant DBN9004.

The phosphinothricin N-acetyltransferase (PAT) gene isolated from *Streptomyces viridochromogenes* catalyzes the conversion of L-phosphinothricin into its inactive form by acetylation to confer plant glufosinate herbicide tolerance. Phosphinothricin (PTC, 2-amino-4-methylphosphonobutyric acid) is an inhibitor of glutamine synthetase. PTC is a structural unit of antibiotic 2-amino-4-methylphosphono-alanyl-alanine, this tripeptide (PTT) has activities against Gram-positive and Gram-negative bacteria and fungus, *Botrytis cinerea.* The phosphinothricin N-acetyltransferase (PAT) gene may also serve as a selectable marker gene.

The "glufosinate" (also known as phosphinothricin) refers to ammonium 2-amino-4-[hydroxy(methyl)phosphonyl]butyrate.Treating with a "glufosinate herbicide" refers to performing treatment using any glufosinate-containing herbicide preparation. The choice of usage rate of a certain glufosinate preparation in order to achieve an effective biological dose will not exceed the skills of an ordinary agronomic technician. Treating a field including plant materials derived from the herbicide-tolerant soybean plant DBN9004 using any glufosinate-containing herbicide preparation will control the growth of weeds in the field and will not affect the growth or yield of the plant materials derived from the herbicide-tolerant soybean plant DBN9004.

The DNA construct is introduced into a plant using a transformation method including, but not limited to, *Agrobacterium*-mediated transformation, gene gun transformation and pollen tube pathway transformation.

The *Agrobacterium*-mediated transformation method is a common method for plant transformation. The exogenous DNA to be introduced into the plant is cloned between the left and right boundary consensus sequences of the vector, i.e., the T-DNA region. An *Agrobacterium* cell is transformed with the vector, and then used to infect a plant tissue, and the T-DNA region of the vector comprising the exogenous DNA is inserted into the plant genome.

The gene gun transformation method is a method of using a carrier containing the exogenous DNA to bombard a plant cell (particle-mediated bio-projectile transformation).

The pollen tube pathway transformation method is a method of carrying the exogenous DNA into embryo sac via nucellus pathway using a natural pollen tube pathway (also called as pollen tube guide tissue) formed by pollination of plants.

After transformation, the transgenic plants must be regenerated from the transformed plant tissue, and the progenies with exogenous DNA are selected using appropriate markers.

The DNA construct is a combination of DNA molecules that are linked to each other, and the combination provides one or more expression cassettes. The DNA construct is preferably a plasmid capable of self-replicating within a bacterial cell and containing different restriction enzyme sites, and the restriction enzyme sites contained therein are used to introduce DNA molecules providing functional gene elements, i.e., a promoter, an intron, a leader sequence, a coding sequence, a 3' terminator region, and other sequences. The expression cassette contained in the DNA construct comprises gene elements which are necessary to provide transcription of the messenger RNA, and the expression cassette can be designed to be expressed in prokaryotic cells or eukaryotic cells. The expression cassettes of the present invention are designed to be most preferably expressed in plant cells.

The transgenic "event" is obtained by transforming a plant cell with a heterologous DNA construct, i.e., comprising generating a plant population by inserting a nucleic acid expression cassette containing a target gene into the plant genome by transgenic methods, regenerating the plant population, and selecting a specific plant having characteristics of the inserted specific genomic sites. The term "event" comprises the original transformant of the heterologous DNA and progenies of this transformant. The term "event" also includes progenies obtained by sexually hybridizing between the transformant and an individual of other species containing heterologous DNA, even if after repeated backcrossing with the backcross parents, the inserted DNA and flanking genomic DNA from the transformant parents are also present at the same chromosomal position in the hybrid progenies. The term "event" also refers to a DNA sequence from an original transformant, the DNA sequence comprises an inserted DNA and a flanking genomic sequence that is closely adjacent to the inserted DNA, and is expected to be transferred to a progeny that is generated by sexually hybridizing a parental line comprising the inserted DNA (e.g., the original transformant and a progeny thereof generated by selfing) with a parental line without the inserted DNA, and the progeny receives the inserted DNA containing the target gene.

"Recombinant" in the present invention refers to the forms of DNA and/or protein and/or organism that is generally not found in nature and thus produced by artificial intervention. Such artificial intervention can produce recombinant DNA molecules and/or recombinant plants. The "recombinant DNA molecule" is obtained by artificially combining two sequence segments that are isolated in other cases, such as nucleic acid segments chemically synthesized or isolated by genetic engineering manipulation techniques. Techniques for performing nucleic acid manipulations are well known.

The term "transgene" comprises any cell, cell line, callus, tissue, plant part or plant, the genotypes thereof will vary due to the presence of a heterologous nucleic acid, and the transgene comprises a transgenic body that is initially altered as such, and a progeny individual derived from the original transgenic bodies by sexual hybridization or asexual reproduction. In the present invention, the term "transgene" does not comprise alterations of genomes (chromosomal or extrachromosomal) caused by conventional plant breeding methods or naturally occurring events, and the naturally occurring events are for example, randomized cross fertilization, non-recombinant viral infection, non-recombinant bacterial transformation, non-recombinant transposition or spontaneous mutation.

In the present invention, "heterologous" refers to a first molecule in nature being generally not found to be combined with a second molecule. For example, a molecule may originate from a first species and be inserted into the genome of a second species. Thus, this molecule is heterologous to the host and is artificially introduced into the genome of the host cell.

Cultivating a transgenic soybean event DBN9004 which is tolerant to a glyphosate herbicide and a glufosinate herbicide is achieved by the following steps: firstly, sexually hybridizing a first parental soybean plant with a second parental soybean plant to produce diverse first filial generation of plants, wherein the first parental soybean plant consists of soybean plants that are cultivated from the transgenic soybean event DBN9004 and its progenies which are obtained by transformation with an expression cassette of the present invention having tolerance to a glyphosate herbicide and a glufosinate herbicide, and the second parental soybean plant lacks tolerance to a glyphosate herbicide and/or a glufosinate herbicide; then selecting the progeny plants that are tolerant to the application of a glyphosate herbicide and/or a glufosinate herbicide, thereby cultivating a soybean plant tolerant to a glyphosate herbicide and a glufosinate herbicides. These steps may further comprise backcrossing a progeny plant having tolerance to the application of the glyphosate herbicide and/or the glufosinate herbicide with the second parental soybean plant or the third parental soybean plant, then selecting the progenies by applying a glyphosate herbicide and a glufosinate herbicide, or by identifying the molecular markers (such as a DNA molecule comprising the conjugation site identified at the 5' terminus and 3' terminus of the inserted sequence in the transgenic soybean event DBN9004) associated with traits, thereby producing a soybean plant that is tolerant to the glyphosate herbicide and the glufosinate herbicide.

It should also to be understood that two different transgenic plants may also be hybridized to produce progenies containing two independent, separately added exogenous genes. The progeny plants which are homozygotes for the two added exogenous genes can be obtained from the selfing of an appropriate progeny. The backcrossing of the parent plants and the outcrossing with non-transgenic plants as described above can also be expected, and the asexual reproduction is also the same.

*Bt* transgenic soybeans can kill, for example, Lepidoptera insects/pests, but there are also a few surviving insects/pests that, after several generations of propagation, may produce resistant insects/pests resistant to Bt protein. In order to address the problem of insect/pest resistance, the US Environmental Protection Agency provides guidance on the use of transgenic crops, i.e., a proportion of shelter soybeans (which may be non-insect resistant transgenic soybeans (which can be non-pest resistant transgenic soybeans (for example, herbicide tolerant transgenic soybeans, or non-target pest resistant transgenic soybeans, or non-transgenic soybeans)) is required to be provided. When most of the insects/pests are killed on the corresponding transgenic insect-resistant soybeans, a portion of the insects/pests are not killed on the shelter soybeans, which ensures that the number of insect/pest population without resistance is dominant. As such, even if a small amount of resistant insects/pests are survived, the resistant genes are significantly diluted after the mating of the resistant insects/pests with the dominant number of non-resistant insects/pests.

The term "probe" is an isolated nucleic acid molecule that binds to a conventional detectable label or reporter molecule thereon, for example, a radioisotope, a ligand, a chemiluminescent agent or enzymes. Such probe is complementary to a strand of the target nucleic acid. In the present invention, the probe is complementary to a DNA strand from the genome of the transgenic soybean event DBN9004, irrespective of the genomic DNA being derived from the transgenic soybean event DBN9004 or seed thereof or derived from the plants or seeds or extracts of the transgene soybean event DBN9004. The probe of the present invention includes not only deoxyribonucleic acid or ribonucleic acid but also polyamides and other probe materials which specifically bind to the target DNA sequence and can be used to detect the presence of the target DNA sequence.

The term "primer" is an isolated nucleic acid molecule that binds to a complementary target DNA strand by nucleic acid hybridization and annealing, forms a hybrid between the primer and the target DNA strand, then extends along the target DNA strand under the action of a polymerase (e.g., DNA polymerase). The primer pairs of the present invention relate to their application in the amplification of target nucleic acid sequences, for example, by polymerase chain reaction (PCR) or other conventional nucleic acid amplification methods.

The lengths of the probe and primer are generally 11 polynucleotides or more, preferably 18 polynucleotides or more, more preferably 24 polynucleotides or more, and most preferably 30 polynucleotides or more. Such probe and primer are specifically hybridized with the target sequence under high stringency hybridization conditions. Although probes that differ from the target DNA sequences and maintain hybridization ability to the target DNA sequences can be designed by conventional methods, it is preferred that the probes and primers of the present invention have complete DNA sequence identity to the consecutive nucleic acids of the target nucleic acids.

Primers and probes of the flanking genomic DNA and insert sequences based on the present invention can be determined by conventional methods, for example, by isolating the corresponding DNA molecule from the plant materials derived from the transgenic soybean event DBN9004 and determining the nucleic acid sequence of the DNA molecule. The DNA molecule comprises a transgenic insert sequence and a flanking sequence of the soybean genome, and a fragment of the DNA molecule can be used as a primer or a probe.

The nucleic acid probes and primers of the present invention hybridize with the target DNA sequence under stringent conditions. Any conventional nucleic acid hybridization or amplification method can be used to identify the presence of DNA derived from the transgenic soybean event DBN9004 in a sample. A nucleic acid molecule or a fragment thereof is capable of specifically hybridizing with other nucleic acid molecules under certain circumstances. As used in the present invention, if two nucleic acid molecules can form an anti-parallel double-stranded nucleic acid structure, then it can be considered that these two nucleic acid molecules can be specifically hybridized with each other. If two nucleic acid molecules exhibit a complete complementarity, then one nucleic acid molecule of the two is said to be the "complement" of the other nucleic acid molecule. As used in the present invention, when each nucleotide of a nucleic acid molecule is complementary to the corresponding nucleotide of another nucleic acid molecule, then these two nucleic acid molecules are the to exhibit a "complete complementarity". If two nucleic acid molecules can be hybridized with each other with a sufficient stability to allow them to anneal and bind with each other at least under conventional "low stringency" conditions, then these two nucleic acid molecules are said to be "minimally complementary". Similarly, if two nucleic acid molecules can be hybridized with each other with a sufficient stability to allow them to anneal and bind with each other under conventional "high stringency" conditions, then these two nucleic acid molecules are said to be "complementary". Deviation from a complete complementarity is permissible, as long as this deviation does not completely prevent two molecules from forming a double-stranded structure. In order to enable a nucleic acid molecule to act as a primer or probe, it is only guaranteed that the molecule has a sufficient complementarity in its sequence to allow a stable double-stranded structure to be formed at the particular solvent and salt concentration employed.

As used in the present invention, a substantially homologous sequence is a segment of a nucleic acid molecule, wherein the nucleic acid molecule can be specifically hybridized with the complementary strand of another segment of a matched nucleic acid molecule under high stringency conditions. Suitable stringent conditions that promote DNA hybridization are for example treating with 6.0x sodium chloride/sodium citrate (SSC) under the condition of about 45°C, and then washing with 2.0× SSC under the condition of 50°C, which conditions are well known to a person skilled in the art. For example, the salt concentration in the washing step may be selected from about 2.0 x SSC at 50°C under low stringency conditions, to about 0.2 x SSC at 50°C under high stringency conditions. In addition, the temperature conditions in the washing step may be increased from about 22°C of ambient temperature under low stringency conditions to about 65°C under high stringency conditions. Both the temperature condition and the salt concentration can be changed, or one of them can be unchanged while the other variable can be changed. Preferably, a nucleic acid molecule of the present invention may be specifically hybridized under moderate stringency conditions, e.g., at about 2.0 x SSC and about 65°C with one or more nucleic acid molecules of SEQ ID NO: 1, SEQ ID NO: 2, SEQ ID NO: 3, SEQ ID NO: 4, SEQ ID NO: 5, SEQ ID NO: 6, and SEQ ID NO: 7 or a complementary sequence thereof, or any of the fragments of the above sequences. More preferably, a nucleic acid molecule of the present invention may be specifically hybridized under high stringency conditions with one or more nucleic acid molecules of SEQ ID NO: 1, SEQ ID NO: 2, SEQ ID NO: 3, SEQ ID NO: 4, SEQ ID NO: 5, SEQ ID NO: 6, and SEQ ID NO: 7 or a complementary sequence thereof, or any of the fragments of the above sequences. In the present invention, the preferred marker nucleic acid molecule has SEQ ID NO: 1, SEQ ID NO: 2, SEQ ID NO: 6 or SEQ ID NO: 7 or a complementary sequence thereof, or any fragment of the above sequences. Another preferred marker nucleic acid molecule of the present invention has 80% to 100%, or 90% to 100% sequence identity to SEQ ID NO: 1, SEQ ID NO: 2, SEQ ID NO: 6 or SEQ ID NO: 7 or a complementary sequence thereof, or any fragment of the above sequences. SEQ ID NO: 1, SEQ ID NO: 2, SEQ ID NO: 6 and SEQ ID NO: 7 can be used as markers in a plant breeding method to identify the progenies of genetic hybridization. The hybridization of a probe with a target DNA molecule can be detected by any method well known to a person skilled in the art including, but not limited to, fluorescence labelling, radioactive labelling, antibody-like labelling, and chemiluminescent labelling.

With respect to amplification (e.g., by PCR) of a target nucleic acid sequence using specific amplification primers, "stringent conditions" refer to conditions that only allow primers to hybridize with the target nucleic acid sequence in a DNA thermal amplification reaction, with a primer of the wild-type sequence (or its complementary sequence) corresponding to the target nucleic acid sequence being capable of binding to the target nucleic acid sequence, and preferably allow the production of an unique amplification product, i.e., the amplicon.

The term "specific binding to (a target sequence)" refers to hybridization of a probe or primer only with a target sequence in a sample containing the target sequence under stringent hybridization conditions.

As used in the present invention, "amplified DNA" or "amplicon" refers to a nucleic acid amplification product of a target nucleic acid sequence that is a part of a nucleic acid template. For example, in order to determine whether a soybean plant is produced by sexual hybridization of the transgenic soybean event DBN9004 of the present invention or whether a soy sample collected from a field contains a transgenic soybean event DBN9004, or whether a soy extract such as soy meal, powder or oils contains the transgenic soybean event DBN9004, the DNA extracted from a soybean plant tissue sample or extract thereof can generate amplicons which are diagnostic for the presence of DNA of the transgenic soybean event DBN9004 by a nucleic acid amplification method using a primer pair. The primer pair comprises a first primer derived from a flanking sequence adjacent to the insertion site of the inserted exogenous DNA in the plant genome and a second primer derived from the inserted exogenous DNA. The amplicon has a length and a sequence that is also diagnostic for the transgenic soybean event DBN9004. The length range of the amplicon may be the binding length of the primer pair plus one nucleotide base pair, preferably plus about 50 nucleotide base pairs, more preferably plus about 250 nucleotide base pairs, most preferably plus about 450 nucleotide base pairs or more.

Optionally, the primer pairs may be derived from flanking genomic sequences on both sides of the inserted DNA to produce an amplicon comprising the entire inserted nucleotide sequence. One of the primer pair derived from the plant genome sequence may be located at a distance from the inserted DNA sequence, and the distance may range from one nucleotide base pair to about 20,000 nucleotide base pairs. The use of the term "amplicon" specifically excludes the primer dimers formed in the DNA thermal amplification reaction.

The nucleic acid amplification reaction can be achieved by any of the nucleic acid amplification methods known in the art, including polymerase chain reaction (PCR). Various nucleic acid amplification methods have been well known to a person skilled in the art. PCR amplification method has been developed to amplify up to 22 kb of genomic DNA and up to 42 kb of bacteriophage DNA. These methods and other DNA amplification methods in the art can be used in the present invention. The genome of the transgenic soybean event DBN9004 can be amplified using the inserted exogenous DNA sequence and the flanking DNA sequence derived from the transgenic soybean event DBN9004 with the provided primer sequences, and standard DNA sequencing of the PCR amplicon or the cloned DNA can be carried out after amplification.

DNA detection kits based on the DNA amplification methods contain DNA primer molecules that specifically hybridize with a target DNA under appropriate reaction conditions and amplify the diagnostic amplicons. The kit can provide a detection method based on the agarose gel or a number of methods known in the art for detecting diagnostic amplicons. A kit comprising a DNA primer that is homologous or complementary to any portion of the soybean genomic region of SEQ ID NO: 3 or SEQ ID NO: 4 and homologous or complementary to any portion of the transgene insertion region of SEQ ID NO: 5 is provided by the present invention. In particular, SEQ ID NO: 8 and SEQ ID NO: 9 are identified as the primer pair useful in the DNA amplification method, and amplify the diagnostic amplicon which is homologous to a portion of the 5' transgene/genomic region of the transgenic soybean event DBN9004, wherein the amplicon comprises SEQ ID NO: 1. Other DNA molecules used as DNA primers may be selected from SEQ ID NO: 5.

The amplicons produced by these methods can be detected by a variety of techniques. One of these methods is Genetic Bit Analysis, in which a DNA oligonucleotide strand that spans the inserted DNA sequence and the adjacent flanking genomic DNA sequence is designed. The oligonucleotide strand is immobilized in microwells of a microwell plate, and after PCR amplification of the target region (using a primer for each of the inserted sequence and the adjacent flanking genomic sequence), the single-stranded PCR product can be hybridized with the immobilized oligonucleotide strand and serve as a template for single base extension reaction which uses DNA polymerase and specifically labelled ddNTPs for the next expected base. The results can be obtained by fluorescence or ELISA-like methods. The signal represents the presence of an inserted/flanking sequence, which indicates that amplification, hybridization, and single base extension reaction are successful.

Another method is the pyrosequencing technique. This method designs an oligonucleotide strand that spans the inserted DNA sequence and the adjacent genomic DNA conjugation site. The oligonucleotide strand is hybridized with the single strand PCR product of the target region (using a primer for each of the inserted sequence and the adjacent flanking genomic sequence), then incubated with DNA polymerase, ATP, sulfurylase, luciferase, apyrase, adenosine-5'-phosphosulfate and luciferin. The dNTPs are added respectively, and the generated optical signals are measured. The optical signal represents the presence of an inserted/flanking sequence, which indicates that amplification, hybridization, and single base or multiple base extension reaction are successful.

The fluorescence polarization phenomenon described by Chen et al. (Genome Res. 9: 492-498, 1999) is also a method that can be used to detect the amplicons of the present invention. The use of this method needs to design an oligonucleotide strand that spans the inserted DNA sequence and the adjacent genomic DNA conjugation site. The oligonucleotide strand is hybridized with the single strand PCR product of the target region (using a primer for each of the inserted sequence and the adjacent flanking genomic sequence), then incubated with DNA polymerase, and a fluorescence labelled ddNTP. Single base extension will result in the insertion of ddNTP. Change in the polarization of such insertion can be measured using a fluorometer. Change in the polarization represents the presence of an inserted/flanking sequence, which indicates that amplification, hybridization, and single base extension reaction are successful.

Taqman is described as a method for the detection and quantitative analysis of the presence of a DNA sequence, and is described in detail in the instructions provided by manufacturer. Now, it is briefly illustrated below, a FRET oligonucleotide probe that spans the inserted DNA sequence and the adjacent genomic flanking conjugation site is designed. The circular reaction is performed using the FRET probe and the PCR primers (using a primer for each of the inserted sequence and the adjacent flanking genomic sequence) in the presence of thermostable polymerases and dNTPs. The hybridization of the FRET probe results in the split of a fluorescent moiety and a quenched moiety on the FRET probe and the release of the fluorescent moiety. The generation of a fluorescent signal represents the presence of an inserted/flanking sequence, which indicates that the amplification and hybridization are successful. Suitable techniques for detecting plant materials derived from the herbicide tolerant transgenic soybean event DBN9004 may also include Southern blot hybridization, Northern blot hybridization, and in situ hybridization based on the hybridization principle. In particular, the suitable technique includes incubating the probe and the sample, washing to remove the unbound probe and detecting whether the probe has been hybridized. The detection method described above depends on the type of marker attached to the probe, for example, a radiolabelled probe can be detected by the exposure and development of an X-ray film, or an enzyme-labelled probe can be detected by achieving the color change through conversion of substrates.

Tyangi et al. (Nat. Biotech. 14: 303-308, 1996) introduced the application of molecular markers in the detection of sequence. It is briefly illustrated below, a FRET oligonucleotide probe that spans the inserted DNA sequence and the adjacent genomic flanking conjugation site is designed. The unique structure of the FRET probe results in its secondary structure, which maintains the fluorescent moiety and the quenched moiety within a cloase distance. The circular reaction is performed using the FRET probe and the PCR primers (using a primer for each of the inserted sequence and the adjacent flanking genomic sequence) in the presence of thermostable polymerases and dNTPs. After the successful PCR amplification, the hybridization of the FRET probe and the target sequence will lead to the loss of the secondary structure of the probe, so that the fluorescent moiety and the quenched moiety are spatially separated to produce a fluorescent signal. The generation of a fluorescent signal represents the presence of an inserted/flanking sequence, which indicates that the amplification and hybridization are successful.

Other described methods, such as microfluidics, provide methods and devices for isolating and amplifying a DNA sample. Luminescent dyes are used to detect and determine specific DNA molecules. A nanotube device comprising an electronic sensor for detecting a DNA molecule or nanobeads to which the specific DNA molecule is bound, which can thus be detected, is useful for detecting the DNA molecule of the present invention.

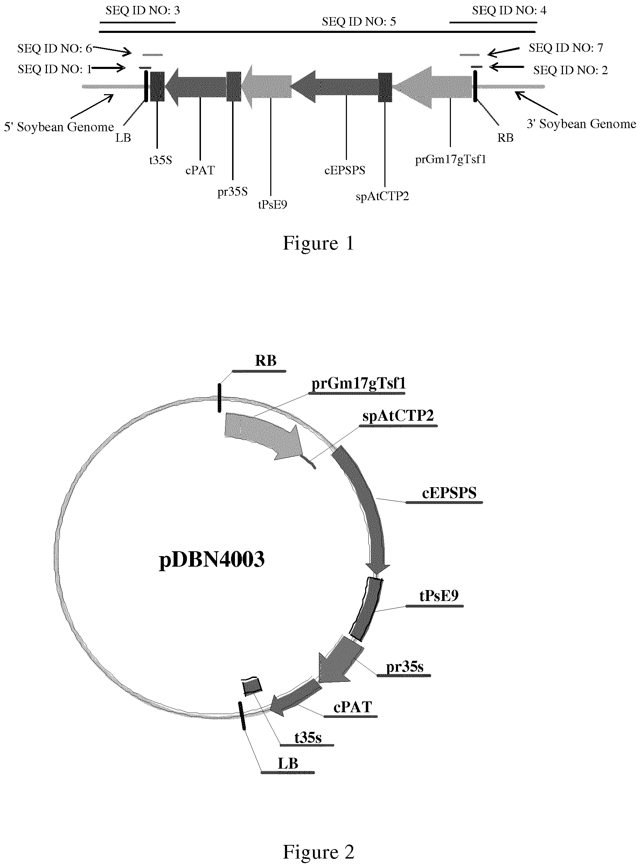

The DNA detection kit can be developed using the compositions described in the present invention and the methods described or known in the field of DNA detection. The kit facilitates the identification of the presence of the DNA of the transgenic soybean event DBN9004 in a sample and can also be used to cultivate the soybean plants containing the DNA of the transgenic soybean event DBN9004. The kit may contain DNA primers or probes homologous to or complementary to at least a portion of SEQ ID NO: 1, 2, 3, 4 or 5 or contain other DNA primers or probes homologous to or complementary to the DNA contained in the transgenic genetic elements of the DNA, and these DNA sequences can be used for DNA amplification reactions or as probes in DNA hybridization methods. The DNA structure, contained in the soybean genome, of the conjugation site of the transgenic inserted sequence and the soybean genome described in Figures 1 and Table 1 comprises: the flanking genomic region of soybean plant DBN9004 located at the 5' terminus of the transgenic inserted sequence; a portion of the inserted sequence from the left boundary region (LB) of the *Agrobacterium*; a first expression cassette consisting of the cauliflower mosaic virus 35S promoter (pr35S) containing a tandem repeat of an enhancer region, which can be operably linked to glufosinate-tolerant phosphinothricin N-acetyltransferase (cPAT) of *Streptomyces* and then operably linked to the cauliflower mosaic virus 35S terminator (t35S); a second expression cassette consisting of the soybean Tsfl gene (encoding elongation factor EF-1α) promoter (prGm17gTsf1), which can be operably linked to the coding sequence (spAtCTP2) of the *Arabidopsis* EPSPS chloroplast transit peptide, then operably linked to glyphosate-tolerant 5-enol-pyruvylshikimate-3-phosphate synthase (cEPSPS) of *Agrobacterium* CP4 strain, and then operably linked to the 3' untranslated sequence (tPse9) derived from pea ribulose-1,5-bisphosphate carboxylase; a portion of the inserted sequence from the right boundary region (RB) of the *Agrobacterium* and the flanking genomic region of soybean plant DBN9004 located at the 3' terminus of the transgenic inserted sequence (SEQ ID NO: 5). In the DNA amplification method, the DNA molecule as a primer may be any portion derived from the transgenic inserted sequence in the soybean plant DBN9004 or may be any portion derived from the DNA region of the flanking soybean genome in the transgenic soybean event DBN9004.

Transgenic soybean Event DBN9004 can be combined with other transgenic soybean varieties such as soybeans with herbicide (eg 2,4-D, dicamba, etc.) tolerance, or transgenic soybean varieties carrying other insect-resistant genes (e.g., *Cry1Ac, Cry2Ab,* etc.). Various combinations of all these different transgenic events, along with the transgenic soybean event DBN9004 of the present invention can be bred together to provide an improved hybrid transgenic soybean variety resistant to various pests and tolerant to various herbicides. These varieties may exhibit superior characteristics such as increased yield compared to a non-transgenic variety and a transgenic variety of single trait.

The present invention provides a nucleic acid sequence for the detection of herbicide tolerant soybean plant DBN9004 and a detection method thereof. The transgenic soybean event DBN9004 is tolerant to phytotoxic effects of glyphosate and/or glufosinate-containing agricultural herbicides. The double-trait soybean plant expresses the glyphosate-resistant 5-enolpyruvylshikimate-3-phosphate synthase (EPSPS) protein of the *Agrobacterium* strain CP4, which confers the plant tolerance to glyphosate, and expresses the glufosinate-resistant phosphinomycin N-acetyltransferase (PAT) protein of *Streptomyces,* which confers the plant tolerance to glufosinate. The double trait soybean has the following advantages: 1) it has the ability of broad-spectrum weed control by applying glyphosate-containing agricultural herbicides to soybean crops; 2) the use of glufosinate herbicides in combination with glufosinate-tolerant traits (mixed or alternated with glyphosate herbicides) can serve as a non-selective means for the effective management of glyphosate-resistant weeds; 3) co-planting herbicide tolerant transgenic soybeans as non insect-resistant transgenic soybeans with transgenic insect-resistant soybeans in a proportion can delay the induction of insect/pest resistance; and 4) there is no reduction in soybean yield. In addition, genes encoding glyphosate-tolerant and glufosinate-tolerant traits are linked on the same DNA segment and are present at a single locus of the genomic soybean DBN9004 genome, which provides enhanced breeding efficiency and enables the use of molecular markers to track the transgenic inserted fragments in the breeding population and progenies thereof. Meanwhile, SEQ ID NO: 1 or its complementary sequence, SEQ ID NO: 2 or its complementary sequence, SEQ ID NO: 6 or its complementary sequence, or SEQ ID NO: 7 or its complementary sequence can be used as DNA primers or probes in the detection method of the present invention to produce an amplification product that is diagnosed as the transgenic soybean event DBN9004 or progenies thereof, and quickly, accurately and stably identify the presence of the plant materials derived from the transgenic soybean event DBN9004.

### Brief Description of Sequence

SEQ ID NO: 1 A sequence with a length of 22 nucleotides at the 5' terminus of the inserted sequence near the insertion conjugation site in the transgenic soybean event DBN9004, wherein nucleotides at positions 1-11 and nucleotides at positions 12-22 locate on both sides of the insertion site on the soybean genome, respectively;
SEQ ID NO: 2 A sequence with a length of 22 nucleotides at the 3' terminus of the inserted sequence near the insertion conjugation site in the transgenic soybean event DBN9004, wherein nucleotides at positions 1-11 and nucleotides at positions 12-22 locate on both sides of the insertion site on the soybean genome, respectively;
SEQ ID NO: 3 A sequence with a length of 1207 nucleotides at the 5' terminus of the inserted sequence near the insertion conjugation site in the transgenic soybean event DBN9004;
SEQ ID NO: 4 A sequence with a length of 631 nucleotides at the 3' terminus of the inserted sequence near the insertion conjugation site in the transgenic soybean event DBN9004;
SEQ ID NO: 5 Entire T-DNA sequence, 5' and 3' flanking soybean genome sequences;
SEQ ID NO: 6 A sequence which is located within SEQ ID NO: 3 and spans the nucleotide sequence in the pDBN4003 construct DNA sequence and t35S transcription termination sequence;
SEQ ID NO: 7 A sequence which is located within SEQ ID NO: 4 and spans the prGm17gTsf1 promoter sequence and the nucleotide sequence in the pDBN4003 construct DNA sequence;
SEQ ID NO: 8 A first primer amplifying SEQ ID NO: 3;
SEQ ID NO: 9 A second primer amplifying SEQ ID NO: 3;
SEQ ID NO: 10 A first primer amplifying SEQ ID NO: 4;
SEQ ID NO: 11 A second primer amplifying SEQ ID NO: 4;
SEQ ID NO: 12 A Primer on 5' flanking genome sequence;
SEQ ID NO: 13 A primer located on T-DNA and paired with SEQ ID NO: 12;
SEQ ID NO: 14 A primer on 3' flanking genome sequence, which is paired with SEQ ID NO: 12 to detect whether the transgene is a homozygote or a heterozygote;
SEQ ID NO: 15 A primer located on T-DNA and paired with SEQ ID NO: 14;
SEQ ID NO: 16 Taqman primer 1 for detecting *EPSPS*;
SEQ ID NO: 17 Taqman primer 2 for detecting *EPSPS*;
SEQ ID NO: 18 Taqman probe 1 for detecting *EPSPS*;
SEQ ID NO: 19 Taqman primer 3 for detecting *PAT*;
SEQ ID NO: 20 Taqman primer 4 for detecting *PAT*;
SEQ ID NO: 21 Taqman probe 2 for detecting *PAT*;
SEQ ID NO: 22 A first primer of soybean endogenous gene of ubiquitin;
SEQ ID NO: 23 A second primer of soybean endogenous gene of ubiquitin;
SEQ ID NO: 24 A probe of *EPSPS* in Southern blot hybridization detection;
SEQ ID NO: 25 A probe of *PAT* in Southern blot hybridization detection;
SEQ ID NO: 26 A primer located on T-DNA in the same direction with SEQ ID NO: 13;
SEQ ID NO: 27 A primer located on T-DNA in the opposite direction to SEQ ID NO: 13 and used to obtain a flanking sequence;
SEQ ID NO: 28 A primer located on T-DNA in the opposite direction to SEQ ID NO: 13 and used to obtain a flanking sequence;
SEQ ID NO: 29 A primer located on T-DNA in the same direction with SEQ ID NO: 15;
SEQ ID NO: 30 A primer located on T-DNA in the opposite direction to SEQ ID NO: 15 and used to obtain a flanking sequence;
SEQ ID NO: 31 A primer located on T-DNA in the opposite direction to SEQ ID NO: 15 and used to obtain a flanking sequence.

The technical solution of the present invention is further described in details through drawings and examples below.

### Description of the Drawings

Figure 1 is a structural representation of the conjugation site between the transgenic inserted sequence and the soybean genome according to the nucleic acid sequence for the detection of herbicide tolerant soybean plant DBN9004 and a detection method thereof of the present invention;
Figure 2 is a structural representation of the recombinant expression vector pDBN4003 according to the nucleic acid sequence for the detection of herbicide tolerant soybean plant DBN9004 and a detection method thereof of the present invention.

### Particular Embodiments

The technical solutions of a nucleic acid sequence for the detection of a herbicide tolerant soybean plant DBN9004 and a detection method thereof according to the present invention will be further illustrated below through the particular embodiments.

### Example 1. Cloning and transformation

### 1.1. Vector cloning

The recombinant expression vector pDBN4003 was constructed using standard gene cloning techniques (as shown in Figure 2). The vector pDBN4003 contains two tandem transgenic expression cassettes, wherein the first expression cassette consists of the soybean Tsfl gene (encoding elongation factor EF-1α) promoter (prGml7gTsfl), which can be operably linked to the coding sequence (spAtCTP2) of the *Arabidopsis* EPSPS chloroplast transit peptide, then operably linked to the glyphosate-tolerant 5-enol-pyruvylshikimate-3-phosphate synthase (cEPSPS) of *Agrobacterium* CP4 strain, and operably linked to the 3' untranslated sequence (tPse9) derived from pea ribulose-1,5-bisphosphate carboxylase; and the second expression cassette consists of the cauliflower mosaic virus 35S promoter (pr35S) containing a tandem repeat of an enhancer region, which can be operably linked to glufosinate-tolerant phosphinothricin N-acetyltransferase (cPAT) of Streptomyces and then operably linked to the cauliflower mosaic virus 35S terminator (t35S).

*Agrobacterium* LBA4404 (Invitrgen, Chicago, USA; Cat. No: 18313-015) was transformed with the vector pDBN4003 by liquid nitrogen method, and the transformed cells were screened using 5-enol-pyruvylshikimate-3-phosphate synthase (EPSPS) as a selective marker.

### 1.2. Plant transformation

The transformation was carried out by conventional *Agrobacterium* infection method, and the sterile cultured soybean cotyledonary node tissue was co-cultured with *Agrobacterium* described in Example 1.1 to introduce the T-DNA in the constructed recombinant expression vector pDBN4003 into the soybean chromosome to produce the transgenic soybean event DBN9004.

As regards the *Agrobacterium*-mediated soybean transformation, briefly, mature soybean seeds were germinated in a soybean germination culture medium (3.1 g/L of B5 salt, B5 vitamin, 20 g/L of sucrose, and 8 g/L of agar, pH 5.6), and the seeds were inoculated in a germination culture medium and cultured under the conditions of: a temperature of 25 ± 1°C; and a photoperiod (light/dark) of 16 h/8 h. After 4-6 days of germination, soybean sterile seedlings swelled at bright green cotyledonary nodes were taken, hypocotyledonary axes were cut off 3-4 millimeters below the cotyledonary nodes, the cotyledons were cut longitudinally, and apical buds, lateral bud and seminal roots were removed. A wound was made at a cotyledonary node using the knife back of a scalpel, the wounded cotyledonary node tissues were contacted with an *Agrobacterium* suspension, wherein the *Agrobacterium* can transfer the nucleotide sequence of *EPSPS* gene and the nucleotide sequence of *PAT* gene to the wounded cotyledonary node tissues (step 1: infection step). In this step, the cotyledonary node tissues were preferably immersed in the *Agrobacterium* suspension (OD₆₆₀ = 0.5-0.8, an infection culture medium (2.15 g/L of MS salt, B5 vitamin, 20 g/L of sucrose, 10 g/L of glucose, 40 mg/L of acetosyringone (AS), 4 g/L of 2-morpholine ethanesulfonic acid (MES), and 2 mg/L of zeatin (ZT), pH 5.3)) to initiate the infection. The cotyledonary node tissues were co-cultured with *Agrobacterium* for a period of time (3 days) (step 2: co-culturing step). Preferably, the cotyledonary node tissues were cultured in a solid culture medium (4.3 g/L of MS salt, B5 vitamin, 20 g/L of sucrose, 10 g/L of glucose, 4 g/L of 2-morpholine ethanesulfonic acid (MES), 2 mg/L of zeatin, and 8 g/L of agar, pH 5.6) after the infection step. After this co-culturing stage, there was an optional "recovery" step. In the "recovery" step, there may be at least one antibiotic (150-250 mg/L of cephalosporin) known to inhibit the growth of *Agrobacterium* in a recovery culture medium (3.1 g/L of B5 salt, B5 vitamin, 1 g/L of 2-morpholine ethanesulfonic acid (MES), 30 g/L of sucrose, 2 mg/L of zeatin (ZT), 8 g/L of agar, 150 mg/L of cephalosporin, 100 mg/L of glutamic acid, and 100 mg/L of aspartic acid, pH 5.6), without the addition of a selective agent for a plant transformant (step 3: recovery step). Preferably, tissue blocks regenerated from the cotyledonary nodes were cultured in a solid culture medium with an antibiotic but without a selective agent, to eliminate *Agrobacterium* and provide a recovery stage for the infected cells. Subsequently, the tissue blocks regenerated from the cotyledonary nodes were cultured in a culture medium containing a selective agent (glyphosate), and growing transformed calli were selected (step 4: selection step). Preferably, the tissue blocks regenerated from the cotyledonary nodes were cultured in a screening solid culture medium (3.1 g/L of B5 salt, B5 vitamin, 1 g/L of 2-morpholine ethanesulfonic acid (MES), 30 g/L of sucrose, 1 mg/L of 6-benzyladenine (6-BAP), 8 g/L of agar, 150 mg/L of cephalosporin, 100 mg/L of glutamic acid, 100 mg/L of aspartic acid, and 10 mg/L of glyphosate isopropylamine salt, pH 5.6) with a selective agent, resulting in continuous growth of transformed cells. Then, plants were regenerated from the transformed cells (step 5: regeneration step). Preferably, the tissue blocks regenerated from the cotyledonary nodes grown in a culture medium containing a selective agent were cultured in solid culture media (B5 differentiation culture medium and B5 rooting culture medium) to regenerate plants.

The screened out resistant tissues were transferred onto the B5 differentiation culture medium (3.1 g/L of B5 salt, B5 vitamin, 1 g/L of 2-morpholine ethanesulfonic acid (MES), 30 g/L of sucrose, 1 mg/L of zeatin (ZT), 8 g/L of agar, 150 mg/L of cephalosporin, 50 mg/L of glutamic acid, 50 mg/L of aspartic acid, 1 mg/L of gibberellin, 1 mg/L of auxin, and 10 mg/L of glyphosate isopropylamine salt, pH 5.6), and cultured at 25°C for differentiation. The differentiated seedlings were transferred onto the B5 rooting culture medium (3.1 g/L of B5 salt, B5 vitamin, 1 g/L of 2-morpholine ethanesulfonic acid (MES), 30 g/L of sucrose, 8 g/L of agar, 150 mg/L of cephalosporin, and 1 mg/L of indole-3-butyric acid (IBA)), cultured in the rooting culture medium to be a height of about 10 cm at 25°C, and transferred to a glasshouse for culturing until fruiting. In the greenhouse, the plants were cultured at 26°C for 16 hours, and then cultured at 20°C for 8 hours every day.

### 1.3. Identification and Screening of Transgenic Events

A total of 288 independent transgenic T₀ plants were produced.

The presence or absence of the *EPSPS* and *PAT* genes in the regenerated transgenic soybean plants was examined by TaqMan™ analysis (see Example 2) and the copy numbers in the glyphosate and glufosinate tolerant strains were characterized. By screening, the event DBN9004 was selected to be excellent in its performance with single-copy transgene, good glyphosate herbicide tolerance, glufosinate herbicide tolerance and agronomic traits (see Example 6).

### Example 2. Detection of the transgenic soybean event DBN9004 by TaqMan.

About 100 mg of leaves were taken from the transgenic soybean event DBN9004 as a sample, and extracted for its genomic DNA using a plant DNA extraction kit (DNeasy Plant Maxi Kit, Qiagen). The copy numbers of the *EPSPS* gene and the *PAT* gene were detected by Taqman probe fluorescence quantitative PCR method. At the same time, wild type soybean plants were used as controls, and detected and analyzed according to the above-mentioned method. Triple repeats were set for the experiments, and averaged.

The specific procedures were as follows:
Step 11. 100 mg of leaves were taken from the transgenic soybean event DBN9004, and milled into a homogenate in a mortar with liquid nitrogen, and triple repeats were set for each sample;
Step 12. Genomic DNAs of the above-mentioned samples were extracted using a DNeasy Plant Mini Kit of Qiagen, and the particular method can refer to the product manual thereof;
Step 13. The concentrations of the genomic DNAs of the above-mentioned samples were detected using NanoDrop 2000 (Thermo Scientific);
Step 14. The concentrations of the genomic DNAs of the above-mentioned samples were adjusted to a consistent concentration value which ranges from 80 to 100 ng/µL;
Step 15. The copy numbers of the samples were identified using the Taqman probe fluorescence quantitative PCR method, wherein samples for which the copy numbers had been identified and known were taken as standards, the samples of the wild type soybean plants were taken as the control, and triple repeats were taken for each sample and averaged; the sequences of fluorescence quantitative PCR primers and a probe were as follows, respectively:
   the following primers and probe were used to detect the *EPSPS* gene sequence:
      primer 1: TTGGTGCTAACCTTACCGTTGAG, shown as SEQ ID NO: 16 in the sequence listing;
      primer 2: GCTTACCACGACCTTCAAGACG, shown as SEQ ID NO: 17 in the sequence listing;
      probe 1: CTGATGCTGACGGTGTGCGTACCATC, shown as SEQ ID NO: 18 in the sequence listing;
   the following primers and probe were used to detect the *PAT* gene sequence:
      primer 3: CAGTTGAGATTAGGCCAGCTACAG, shown as SEQ ID NO: 19 in the sequence listing;
      primer 4: TTCACTGTAGACGTCTCAATGTAATGG, shown as SEQ ID NO: 20 in the sequence listing;
      probe 2: CAGCTGATATGGCCGCGGTTTGTG, shown as SEQ ID NO: 21 in the sequence listing;

**PCR reaction system:**

| | |
|---|---|
| JumpStart™ Taq ReadyMix™ (Sigma) | 10 µL |
| 50 × primer/probe mixture | 1 µL |
| genomic DNA | 3 µL |
| water (dd H2O) | 6 µL |

The 50 × primer/probe mixture comprises 45 µL of each primer at a concentration of 1 mM, 50 µL of the probe at a concentration of 100 µM, and 860 µL of L1× TE buffer, and was stored at 4°C in an amber tube.

**PCR reaction conditions:**

| Step | Temperature | Time |
|---|---|---|
| 21 | 95°C | 5 minutes |
| 22 | 95°C | 30 seconds |
| 23 | 60°C | 1 minutes |
| 24 | back to step 22, repeated 40 times | |

The data were analyzed using SDS 2.3 software (Applied Biosystems), and the single-copy transgenic soybean event DBN9004 was obtained.

### Example 3. Detection of the transgenic soybean event DBN9004

### 3.1. Extraction of Genomic DNA

DNA extraction was carried out according to the conventionally used CTAB (cetyltrimethylammonium bromide) method: taking 2 g of young leaves from the transgenic soybean event DBN9004, and milling into powder in the liquid nitrogen, followed by adding 0.5 mL of DNA extraction CTAB buffer (20 g/L of CTAB, 1.4 M NaCl, 100 mM Tris -HCl, 20 mM EDTA (ethylenediaminetetraacetic acid), with pH adjusted to 8.0 with NaOH) preheated at a temperature of 65°C, and thoroughly mixing and then extracting at a temperature of 65°C for 90 minutes; adding 0.5-fold volume of phenol and 0.5-fold volume of chloroform, then inverting for mixing uniformly; centrifuging at 12000 rpm (revolutions per minute) for 10 minutes; pipetting the supernatant, 2-fold volume of anhydrous ethanol, gently shaking the centrifuge tube, and standing at a temperature of 4°C for 30 minutes; centrifuging at a rotational speed of 12000 rpm for 10 minutes again; collecting the DNA into the bottom of the tube; discarding the supernatant and washing the precipitate with 1 mL ethanol with a mass concentration of 70%; centrifuging at a rotational speed of 12000 rpm for 5 minutes; suction-drying under vacuum or blow-drying on a super clean bench; and dissolving DNA precipitate in an appropriate amount of TE buffer (10 mM Tris-HCl, 1 mM EDTA, pH 8.0) and storing at a temperature condition of -20°C.

### 3.2. Analysis of Flanking DNA Sequence

The concentration of the above extracted DNA sample was measured so that the concentration of the sample to be tested was between 80-100 ng/µL. Genomic DNA was digested with the selected restriction enzymes *Psi*I*, Dra*I and *Taq*I (for 5' terminus analysis) and *Afl*II*, Taq*I and *Psi*I (for 3' terminus analysis), respectively. 26.5 µL of genomic DNA, 0.5 µL of the above selected restriction enzymes and 3 µL of enzyme digestion buffer were added into each enzyme digestion system, and digested for 1 hour. After the completion of enzyme digestion, 70 µL of absolute ethanol was added to the enzyme digestion system, then put into an ice-bath for 30 minutes, centrifuged at a rotational speed of 12000 rpm for 7 minutes, the supernatant was discarded, blow-dried, after which 8.5 µl of double distilled water (dd H₂O), 1 µL 10X T₄-DNA ligase buffer (NEB T4 DNA Ligase Reaction Buffer, and its specific formula can refer to the NEB website or https://www.neb.com/products/restriction-endonucleases and https://www.neb.com/products/b0202-t4-dna-ligase-reaction-buffer) and 0.5 µL T₄-DNA ligase were added for ligation at a temperature of 4°C overnight. The PCR amplification was carried out with a series of nested primers to isolate 5' and 3' transgene/genomic DNAs. Specifically, the primer combination for isolating 5' transgene/genomic DNA comprises SEQ ID NO: 13 and SEQ ID NO: 26 as a first primer, SEQ ID NO: 27 and SEQ ID NO: 28 as a second primer, and SEQ ID NO: 13 as a sequencing primer. Specifically, the primer combination for isolating 3' transgene/genomic DNA comprises SEQ ID NO: 15 and SEQ ID NO: 29 as a first primer, SEQ ID NO: 30 and SEQ ID NO: 31 as a second primer, and SEQ ID NO: 15 as a sequencing primer, and PCR reaction conditions were shown as in Table 3.

The obtained amplicons were electrophoresed on a 2.0% agarose gel to separate the PCR reaction product, subsequently the target fragments were isolated from the agarose matrix using a gel recovery kit (QIAquick Gel Extraction Kit, Cat. No. 28704, Qiagen Inc., Valencia, CA). Then, the purified PCR product was sequenced (e.g., ABI PrismTM 377, PE Biosystems, Foster City, CA) and analyzed (e.g., DNASTAR sequence analysis software, DNASTAR Inc., Madison, WI).

The 5' and 3' flanking sequences and contact sequences were identified using a standard PCR method. The 5' flanking sequence and contact sequence can be identified using SEQ ID NO: 8 or SEQ ID NO: 12, in combination with SEQ ID NO: 9, SEQ ID NO: 13, or SEQ ID NO: 26. The 3' flanking sequence and contact sequence can be identified using SEQ ID NO: 11 or SEQ ID NO: 14, in combination with SEQ ID NO: 10, SEQ ID NO: 15, or SEQ ID NO: 29. The PCR reaction system and the amplification conditions were shown as in Table 2 and Table 3. A person skilled in the art would appreciate that other primer sequences can also be used to identify flanking sequences and contact sequences.

DNA sequencing of the PCR product provides DNA that can be used to design other DNA molecules which can be used as primers and probes for the identification of soybean plants or seeds derived from the transgenic soybean event DBN9004.

It was found that the positions 1-1268 of the nucleotides of SEQ ID NO: 5 were shown to be the right boundary flank (5' flanking sequence) of the transgenic soybean event DBN9004 inserted sequence in the soybean genome sequence, and the positions 6068-7059 of the nucleotides of SEQ ID NO: 5 were shown to be the left boundary flank (3' flanking sequence) of the transgenic soybean event DBN9004 inserted sequence in the soybean genome sequence. 5' conjugating sequence was listed in SEQ ID NO: 1, and 3' conjugating sequence was listed in SEQ ID NO: 2.

### 3.3. PCR conjugation assay

The conjugating sequence is a relatively short polynucleotide molecule, which is a novel DNA sequence that is diagnostic for DNA of the transgenic soybean event DBN9004 when the conjugating sequence is detected in a polynucleic acid assay. The conjugating sequences in SEQ ID NO: 1 and SEQ ID NO: 2 are 11 polynucleotides at each side of the insertion site of the transgenic fragments and the soybean genomic DNA in the transgenic soybean event DBN9004. A longer or shorter polynucleotide conjugating sequence may be selected from SEQ ID NO: 3 or SEQ ID NO: 4. The conjugating sequences (5' linking region SEQ ID NO: 1, and 3' linking region SEQ ID NO: 2) are useful as DNA probes or DNA primer molecules in the DNA detection methods. The conjugating sequences SEQ ID NO: 6 and SEQ ID NO: 7 are also novel DNA sequences in the transgenic soybean event DBN9004, and can also be used as DNA probes or DNA primer molecules to detect the presence of transgenic soybean event DBN9004 DNA. The SEQ ID NO: 6 (positions 741-872 of nucleotides of SEQ ID NO: 3) spans the pDBN4003 construct DNA sequence and t35S terminator sequence, and the SEQ ID NO: 7 (positions 170-287 of nucleotides of SEQ ID NO: 4) spans the prGm17gTsf1 promoter sequence and pDBN4003 construct DNA sequence.

In addition, an amplicon is generated using at least one primer from SEQ ID NO: 3 or SEQ ID NO: 4, and the primer when used in the PCR method is used for generating a diagnostic amplicon of the transgenic soybean event DBN9004.

Specifically, a PCR product is generated from the 5' terminus of the transgenic inserted sequence, and is a portion of the genomic DNA that flanks the 5' terminus of the T-DNA inserted sequence in the genome containing the plant materials derived from the transgenic soybean event DBN9004. This PCR product comprises SEQ ID NO: 3. In order to perform the PCR amplification, primer 5 (SEQ ID NO: 8) hybridized with the genomic DNA sequence flanking the 5' terminus of the transgenic inserted sequence, and primer 6 (SEQ ID NO: 9) which is paired with primer 5 and located at the transgenic t35S transcription termination sequence are designed.

A PCR product is generated from the 3' terminus of the transgenic inserted sequence, and comprises a portion of the genomic DNA that flanks the 3' terminus of the T-DNA inserted sequence in the genome containing the plant materials derived from the transgenic soybean event DBN9004. This PCR product comprises SEQ ID NO: 4. In order to perform the PCR amplification, primer 8 (SEQ ID NO: 11) hybridized with the genomic DNA sequence flanking the 3' terminus of the transgenic inserted sequence, and primer 7 (SEQ ID NO: 10), which is paired with primer 8 and located at the 3' terminus of insert, of the prGm17gTsf1 promoter sequence.

The DNA amplification conditions described in Tables 2 and 3 can be used for the above-mentioned PCR conjugation to produce a diagnostic amplicon of the transgenic soybean event DBN9004. The detection of amplicons can be carried out using Stratagene Robocycler, MJ Engine, Perkin-Elmer 9700 or Eppendorf Mastercycler Gradien thermocycler, etc., or by methods and devices known to a person skilled in the art.

**Table 2. PCR steps and reaction mixture conditions for the identification of 5' transgenic insert/genomic conjugation region of the transgenic soybean event DBN9004**

| Steps | Reagents | Amount | Notes |
|---|---|---|---|
| 1 | Nuclease-free water | which is added to a final volume of 20 µL | |
| 2 | 10 × reaction buffer (with MgCl₂) | 2.0 µL | A final concentration of 1 × buffer, and MgCl₂ with a final concentration of 1.5 mM |
| 3 | 10 mM solution of dATP, dCTP, dGTP and dTTP | 0.4 µL | Each dNTP with a final concentration of 200 µM |
| 4 | Event primer 5 (SEQ ID NO: 8 resuspended in 1 × TE buffer or nuclease-free water, to a concentration of 10 µM) | 0.2 µL | A final concentration of 0.1 µM |
| 5 | Event primer 6 (SEQ ID NO: 9 resuspended in 1 × TE buffer or nuclease-free water, to a concentration of 10 µM) | 0.2 µL | A final concentration of 0.1 µM |
| 6 | RNase, free of DNase (500 µg/ml) | 0.1 µL | 50 ng/reaction |
| 7 | *REDTaq*® DNA Polymerase (1 unit/µl) | 1.0 µL (it is suggested to switch the pipet before the next step) | 1 unit/reaction |
| 8 | Extracted DNA (template): the leaves of the sample to be analyzed | 200 ng genomic DNA | |
| | Negative control | 50 ng non-transgenic soybean genomic DNA | |
| | Negative control | Template-free DNA (a solution in which DNAs were resuspended) | |
| | Positive control | 50 ng soybean genomic DNA comprising DBN9004 | |

**Table 3. Perkin-Elmer 9700 thermocycler conditions**

| Number of cycle | Setting |
|---|---|
| 1 | 94°C for 3 minutes |
| 34 | 94°C for 30 seconds |
| | 64°C for 30 seconds |
| | 72°C for 1 minutes |
| 1 | 72°C for 10 minutes |

Materials were gently mixed, if there was no insulation cap on the thermocycler, 1-2 drops of mineral oil could be added above each reaction solution. PCR was carried out using the above cycle parameters (Table 3) on Stratagene Robocycler (Stratagene, La Jolla, CA), MJ Engine (MJ R-Biorad, Hercules, CA) Perkin-Elmer 9700 (Perkin Elmer, Boston, MA) or Eppendorf Mastercycler Gradient (Eppendorf, Hamburg, Germany) thermocycler. MJ Engine or Eppendorf Mastercycler Gradient thermocycler should be run in the calculation mode. The ramp speed of temperature should be set to the maximum value when running the Perkin-Elmer 9700 thermocycler.

The experimental results suggested that: When primers 5 and 6 (SEQ ID NOs: 8 and 9) were used in the PCR reaction of the genomic DNA of the transgenic soybean event DBN9004, an amplification product of the 1207 bp fragment will be produced; when the primers were used in the PCR reactions of the untransformed soybean genomic DNA and non-DBN9004 soybean genomic DNA, no fragment will be amplified; When primers 7 and 8 (SEQ ID NOs: 10 and 11) were used in the PCR reaction of the genomic DNA of the transgenic soybean event DBN9004, an amplification product of the 631 bp fragment will be produced; when the primers were used in the PCR reactions of the untransformed soybean genomic DNA and non-DBN9004 soybean genomic DNA, no fragment will be amplified.

PCR conjugation assay can also be used to identify whether a material derived from the transgenic soybean event DBN9004 is a homozygote or a heterozygote. Primer 9 (SEQ ID NO: 12), primer 10 (SEQ ID NO: 13) and primer 11 (SEQ ID NO: 14) were used for the amplification reaction to produce a diagnostic amplicon of the transgenic soybean event DBN9004. The DNA amplification conditions described in Tables 4 and 5 can be used for the above-mentioned conjugation assay to produce a diagnostic amplicon of the transgenic soybean event DBN9004.

**Table 4. Reaction solution of the conjugation assay**

| Steps | Reagents | Amount | Notes |
|---|---|---|---|
| 1 | Nuclease-free water | which is added to a final volume of 10 µL | |
| 2 | 2 × Universal Master Mix (Applied Biosystems Cat. No. 4304437) | 5 µL | 1 × final concentration |
| 3 | Primer 9 SEQ ID NO: 12, primer 10 SEQ ID NO: 13 and primer 11 SEQ ID NO: 14 (resuspended in nuclease-free water to a concentration of 10 µM) | 0.3 µL | A final concentration of 0.1 µM |
| 4 | *REDTaq*® DNA Polymerase (1 unit/µl) | 1.0 µL (it is suggested to switch the pipet before the next step) | 1 unit/reaction |
| 5 | Extracted DNA (template): the leaves of the sample to be analyzed | 200 ng genomic DNA | |
| | Negative control | 50 ng non-transgenic soybean genomic DNA | |
| | Negative control | Template-free DNA (a solution in which DNAs were resuspended) | |
| | Positive control | 50 ng of soybean genomic DNA containing DBN9004 | |

**Table 5. Perkin-Elmer 9700 thermocycler conditions of the conjugation assay**

| Number of cycle | Setting |
|---|---|
| 1 | 95°C for 10 minutes |
| 10 | 95°C for 15 seconds |
| | 64°C for 1 minute (-1°C /cycle) |
| 30 | 95°C for 15 seconds |
| | 54°C for 1 minutes |
| 1 | Immersion at 10°C |

PCR was carried out using the above cycle parameters (Table 5) on Stratagene Robocycler (Stratagene, La Jolla, CA), MJ Engine (MJ R-Biorad, Hercules, CA) Perkin-Elmer 9700 (Perkin Elmer, Boston, MA) or Eppendorf Mastercycler Gradient (Eppendorf, Hamburg, Germany) thermocycler. MJ Engine or Eppendorf Mastercycler Gradient thermocycler should be run in the calculation mode. The ramp speed of temperature should be set to the maximum value when running the Perkin-Elmer 9700 thermocycler.

In the amplification reaction, the biological sample containing the template DNA contains DNA for diagnosing the presence of the transgenic soybean event DBN9004 in the sample; or the reaction will produce two different DNA amplicons from the biological sample containing DNA derived from the soybean genome, the DNA derived from the soybean genome is heterozygous with respect to the allele corresponding to the inserted DNA present in the transgenic soybean event DBN9004. These two different amplicons will correspond to a first amplicon derived from the wild-type soybean genome locus and a second amplicon that diagnoses the presence of the DNA of transgenic soybean event DBN9004. Only the soybean DNA sample corresponding to a single amplicon of the second amplicon described for the heterozygous genome is produced, the presence of the transgenic soybean event DBN9004 in the sample can thus be diagnosed and determined, and the sample is produced by the soybean seeds which are homozygous with respect to the allele corresponding to the inserted DNA present in the transgenic soybean plant DBN9004.

It should be noted that the primer pairs of the transgenic soybean event DBN9004 were used to generate amplicons which are diagnostic for the genomic DNA of the transgenic soybean event DBN9004. These primer pairs include but are not limited to, primers 5 and 6 (SEQ ID NOs: 8 and 9), and primers 7 and 8 (SEQ ID NOs: 10 and 11) for use in the DNA amplification method described above. In addition, one control primer pair of primers 12 and 13 (SEQ ID NOs: 22 and 23) used to amplify the soybean endogenous gene was included as an internal standard for the reaction conditions. The analysis of the DNA extraction sample of the transgenic soybean event DBN9004 should include a positive tissue DNA extract control of the transgenic soybean event DBN9004, a negative DNA extract control derived from the non-transgenic soybean event DBN9004 and a negative control free from the template soybean DNA extract. In addition to these primer pairs, any of the primer pairs from SEQ ID NO: 3 or SEQ ID NO: 4, or complementary sequences thereof, can also be used, and when they are used in the DNA amplification reactions, amplicons containing SEQ ID NO: 1 or SEQ ID NO: 2 which are diagnostic with respect to the tissues derived from transgenic event soybean plant DBN9004 will be produced, respectively. The DNA amplification conditions described in Tables 2-5 can be used to produce diagnostic amplicons of the transgenic soybean event DBN9004 with suitable primer pairs. When tested in the DNA amplification method, the extract containing DNA of the soybean plant or seeds which produces an amplicon diagnostic for the transgenic soybean event DBN9004 and is thus presumed to comprise the transgenic soybean event DBN9004, or the product derived from the transgenic soybean event DBN9004, can be used as a template for amplification to determine the presence of the transgenic soybean event DBN9004.

Example 4. Detection of the transgenic soybean event DBN9004 by Southern blot hybridization

### 4.1. DNA extraction for Southern blot hybridization

Southern blot analysis was performed using T4, T5 and T6 generation homozygous transformed events. The plant tissue was milled in liquid nitrogen using a mortar and a pestle. 4-5 g of the milled plant tissue was resuspended in 20 mL of CTAB lysis buffer (100 mM Tris pH 8.0, 20 mM EDTA pH 8.0, 1.4 M NaCl, 0.2% v/v β-mercaptoethanol, 2% w/v polyvinyl-pyrrolidone), and incubated at a temperature of 65°C for 60 minutes. During the incubation period, the sample was inverted for mixing uniformly once every 10 minutes. After incubation, an equal volume of chloroform/isoamyl alcohol (24 : 1) was added, and gently mixed by inverting, then centrifuged at a rotational speed of 4000 rpm for 20 minutes. The aqueous phase was collected, and stood at a temperature of -20°C for 1 hour after the addition of an equal volume of isopropanol, so as to precipitate DNA, then centrifuged at a rotational speed of 4000 rpm for 5 minutes to obtain the DNA precipitate which was then resuspended in 1 mL of TE buffer. In order to degrade any existing RNA, the DNA was incubated at a temperature of 37°C for 30 minutes with 5 µL of RNAase A with a concentration of 30 mg/mL, then centrifuged at a rotational speed of 4000 rpm for 5 minutes, and then centrifuged at a rotational speed of 14,000 rpm for 10 minutes to precipitate DNA in the presence of 0.1 volume of 3 M sodium acetate and 2 volumes of anhydrous ethanol. After the supernatant was discarded, the precipitate was washed with 1 mL of 70% (v/v) ethanol, dried, and then re-dissolved in 1 mL of TE buffer. The concentrations of the genomic DNAs of the above-mentioned samples were detected using an ultramicrospectrophotometer (NanoDrop 2000, Thermo Scientific).

### 4.2. Digestion with restriction enzymes

In a 100 µL reaction system, 5 µg of DNA was digested each time. Genomic DNA was digested with restriction enzymes *Eco*RI and *EcoRV,* respectively, with partial sequences of *EPSPS* and *PAT* on T-DNA as probes. For each enzyme, the digestion products were incubated overnight at an appropriate temperature. The sample was rotated with a centrifugal vacuum concentrator (speed vacuum, Thermo Scientific) to reduce the volume to 20 µL.

### 4.3. Gel electrophoresis

Bromophenol blue loading dye was added to each sample from Example 4.2, and each sample was loaded onto 0.7% agarose gel containing ethidium bromide, then separated by electrophoresis in TAE electrophoresis buffer (40 mM Tris-acetic acid, 2 mM EDTA, pH 8.0), and the gel electrophoresis was run overnight at 20 volts.

The gel was treated with a denaturation solution (1.5 M NaCl, 0.5 M NaOH) and a neutralization solution (1.5 M NaCl, 0.5 M Tris, pH 7.2) for 30 mins, respectively. 5 × SSC was poured into a porcelain dish, a piece of glass was covered, and then a wet filter paper bridge, a gel, a positively charged nylon film (Roche, Cat. No. 11417240001), a three-layer filter paper, a paper tower, and a heavy object were placed successively. After trans-blotting overnight at room temperature, the nylon membrane was rinsed in 2 × SSC for 10 seconds, and the DNA was immobilized on the membrane by UV cross-linking.

### 4.4. Hybridization

The appropriate DNA sequence was amplified by PCR for probe preparation. The DNA probes are SEQ ID NO: 24 and SEQ ID NO: 25, or are homologous or complementary to the above sequences. The DIG labelling of probes, southern blot hybridization, signal detection, and other operations were performed using the DNA high-efficient digoxin labelling and detection kit II (DIG High Prime DNA labelling and Detection Starter Kit II kit, Roche, Cat. No. 1585614910), and the specific methods can refer to their product specifications.

Each Southern contains two control samples: (1) DNA from a negative (non-transformed) segregant, which is used to identify any endogenous soybean sequence that can hybridize to an element-specific probe; (2) a *Hin*d III-digested pDBN4003 plasmid equivalent to one copy number based on a probe length,which is used as a positive control for hybridization and used to illustrate the sensitivity of the experiment.

The hybridization data provided corroborative evidence to support TaqMan™ PCR analysis, i.e., the soybean plant DBN9004 contains single copies of the *EPSPS* and *PAT* genes. Single bands of about 11 kb and 13 kb were generated by the digestion of *Eco*R I and *Eco*R V using the EPSPS probe, respectively; single bands of about 8 kb and 3.2 kb were generated by the digestion of *Eco*R I and *Eco*R V using the PAT probe, respectively. This indicated that each copy of *EPSPS* and *PAT* are present in the soybean transformed event DBN9004.

### Example 5. Detection of the transgenic soybean event named protein by ELISA

The expression range of EPSPS and PAT proteins in the transgenic soybean event DBN9004 can be detected by ELISA.

2 mg of fresh leaves of transgenic soybean event DBN9004 were taken as the sample, and milled in liquid nitrogen, followed by adding 1 mL of the extract buffer (8 g/L NaCl, 0.27 g/L KH₂PO₄, 1.42 g/L Na₂HPO₄, 0.2 g/L KCl, and 5.5 ml/L of Tween-20, pH 7.4), then centrifuging at a rotational speed of 4000 rpm for 10 minutes, diluting the supernatant 40-fold with the extract buffer and taking 80 µl of the diluted supernatant for ELISA detection.

The proportions of the amounts of proteins (EPSPS protein and PAT protein) in the sample by weight of the fresh leaves were measured and analyzed by ELISA (enzyme linked immunosorbent assay) kits (ENVIRLOGIX Company, EPSPS kit and PAT kit). The specific methods can refer to their product specifications. At the same time, leaves of wild type soybean plants (non-transgenic, NGM) were used as controls, and detected and analyzed according to the above-mentioned method, and sextuple repeats were set for each plant.

The experimental results of the contents of proteins (EPSPS protein and PAT protein) of the transgenic soybean event DBN9004 are as shown in Table 6. The proportions of average expression quantities of EPSPS proteins in fresh leaves of transgenic soybean event DBN9004 and wild type soybean plants by weight of fresh leaves (µg/g) were determined to be 230.9 and 0, respectively; and the proportions of average expression quantities of PAT proteins in fresh leaves of transgenic soybean event DBN9004 and wild type soybean plants by weight of fresh leaves (µg/g) were 200.1 and 0, respectively.

**Table 6. Average measurement results of expression quantities (µg/g) of proteins of transgenic soybean event DBN9004**

| | | |
|---|---|---|
| Protein/plant | DBN9004 | NGM |
| EPSPS protein | 230.9 ± 10.4 | 0 |
| PAT protein | 200.1 ± 10.7 | 0 |

### Example 6. Detection of the herbicide tolerance of transgenic soybean event DBN9004

In this experiment, Roundup herbicide (41% glyphosate isopropylammonium salt aqueous agent) and Basta herbicide (with an active ingredient of 18% glufosinate) were used to spray. Random block design was used, with 3 replicates. The area of the plot is 18 m² (5 m × 3.6 m), with a row spacing of 60 cm, a plant spacing of 8 cm, the conventional cultivation and management are used, and there is a wide isolation zone of 1 m between plots. The following 3 types of treatment were performed on the transgenic soybean event DBN9004: 1) no herbicide was sprayed, and the weeds were artificially controlled; 2) Roundup herbicide was sprayed at a dose of 1680 g a.e./ha. (a.e./ha refers to the "active ingredient acid equivalent per hectare") during the V3 leaf stage, then Roundup herbicide was sprayed again during the R2 stage (full-bloom stage) at the same dose; 3) Basta herbicide was sprayed at a dose of 800 g a.i./ha. (a.i./ha refers to the "active ingredient per hectare") during the V3 leaf stage, then Basta herbicide was sprayed again during the V6 stage at the same dose; 4) Basta herbicide was sprayed at a dose of 800 g a.i./ha during the V3 leaf stage, then Roundup herbicide was sprayed at a dose of 1680 g a.e./ha during the R2 stage. A parallel control experiment was carried out with wild-type soybean plants (non-transgenic, NGM). It should be noted that the conversion of glyphosate herbicides of different contents and dosage forms to the form of glyphosate acid equivalent, and the conversion of glufosinate solutions of different concentrations to the active ingredient glufosinate equivalent described above applied to the following conclusions.

The symptoms of phytotoxicity were investigated at 1 week and 2 weeks post administration, and the yield of soybean in the plot was measured when harvesting. Grading of the phytotoxicity symptoms was shown as in Table 7. The herbicide damage rate was used as an index to evaluate the herbicide tolerance of the transformed event. Specifically, the herbicide damage rate (%) = ∑ (number of damaged plants of the same level × level number) / (total number of plants × the highest level); wherein the herbicide damage rate includes the glyphosate damage rate of and the glufosinate damage rate, and the herbicide damage rate was determined based on the investigation results of phytotoxicity 2 weeks post treatment with glyphosate or glufosinate. The yield of soybean in each plot is the total yield (weight) of the soybean grains weighed in middle three rows of each plot. The difference in yield between the different treatments is measured in a form of yield percentage, wherein yield percentage (%) = yield with spraying/yield without spraying. The results of herbicide tolerance of transgenic soybean event DBN9004 and yield of soybean were as shown in Table 8.

**Table 7. Grading standards of the phytotoxicity degree caused by herbicides to soybeans**

| Phytotoxicity level | Symptom description |
|---|---|
| 1 | growing normally, without any damage symptoms |
| 2 | mild phytotoxicity, less than 10% of phytotoxicity |
| 3 | moderate phytotoxicity, able to recover later, not affecting yield |
| 4 | relatively severe phytotoxicity, difficult to recover, resulting in reduced yield |
| 5 | severe phytotoxicity, unable to recover, resulting in significantly reduced yield or total loss of yield |

**Table 8. The results of herbicide tolerance of transgenic soybean event DBN9004 and results of yield test**

| Item/plant | DBN9004 | NGM |
|---|---|---|
| Herbicide damage rate (%) (without spraying) | 0 | 0 |
| Glyphosate damage rate (%) | 0 | 100 |
| Glufosinate damage rate (%) | 0 | 100 |
| Glufosinate + glyphosate damage rate (%) | 0 | 100 |
| Yield percentage (%) (glyphosate) | 109.7 | 0 |
| Yield percentage (%) (glufosinate) | 106.2 | 0 |
| Yield percentage (%) (glufosinate + glyphosate) | 111.5 | 0 |

The results showed that as regards the herbicide (glyphosate and glufosinate) damage rate: 1) the damage rate of transgenic soybean event DBN9004 was substantially 0 under the treatment of glyphosate herbicide (1680 g a.e./ha); 2) the damage rate of transgenic soybean event DBN9004 was also substantially 0 under the treatment of glufosinate herbicide (800 g a.i./ha); 3) the damage rate of transgenic soybean event DBN9004 was also substantially 0 under the treatment of glufosinate herbicide (800 g a.i./ha) and glyphosate herbicide (1680 g a.e./ha); thus, the transgenic soybean event DBN9004 has good herbicide (glyphosate and glufosinate) tolerance.

As regards the yield: the yield of the transgenic soybean event DBN9004 was slightly increased under three treatments of spraying with glyphosate herbicide (1680 g a.e./ha), glufosinate herbicide (800 g a.i./ha), and glufosinate herbicide (800 g a.i./ha) + glyphosate herbicides (1680 g a.e./ha) compared with the treatment without spraying, thereby further indicating that the transgenic soybean event DBN9004 had good herbicide (glyphosate and glufosinate) tolerance.

### Example 7.

For example, agricultural products or commodities can be produced from the transgenic soybean event DBN9004. If sufficient expression quantity is detected in the agricultural products or commodities, the agricultural products or commodities are expected to contain a nucleotide sequence capable of diagnosing the presence of the transgenic soybean event DBN9004 material in the agricultural products or commodities. The food or commodities include, but are not limited to, products derived from soybean plant DBN9004, such as soybean cakes, powders and oils, specifically lecithins, fatty acids, glycerol, sterols, edible oils, defatted soy flakes, defatted and baked soy flours, soy milk clots, bean curd, soy protein concentrates, isolated soy proteins, hydrolyzed vegetable proteins, organized soy proteins, soy protein fibers, etc. A nucleic acid detection method and/or kit based on a probe or primer pair can be developed to detect a nucleotide sequence of the transgenic soybean event DBN9004 as shown in for example, SEQ ID NO: 1 or SEQ ID NO: 2 in a biological sample, wherein the probe sequence or primer sequence is selected from the sequences as shown in SEQ ID NO: 1, SEQ ID NO: 2, SEQ ID NO: 3, SEQ ID NO: 4 and SEQ ID NO: 5 or a fragment thereof or a complementary sequence thereof, to diagnose the presence of transgenic soybean event DBN9004.

In conclusion, the transgenic soybean event DBN9004 of the present invention has better tolerance to a glyphosate herbicide and a glufosinate herbicide and has no influence on the yield and the detection method can accurately and quickly identify whether a DNA molecule of the transgenic soybean event DBN9004 is contained in a biological sample.

The seeds corresponding to the transgenic soybean event DBN9004 had been preserved in the China General Microbiological Culture Collection Center (abbreviated as CGMCC, address: Institute of Microbiology, Chinese Academy of Sciences, No. 3, No. 1 Courtyard, West Beichen Road, Chaoyang District, Beijing, Postcode: 100101) on November 27, 2015, with classification and nomenclature being soybean (*Glycine max*), and an accession number being CGMCC No. 11171. The collections will be kept in the depository for 30 years.

Finally, it should be stated that the above embodiments are merely used for illustrating rather than limiting the technical solution of the present invention; and although the present invention has been described in detail with reference to the preferred embodiments, a person skilled in the art should understand that modifications or equivalent substitutions may be made to the technical solution of the present invention without departing from the spirit and scope of the technical solution of the present invention.

## Claims

1. A nucleic acid molecule having the following nucleic acid sequence, wherein the nucleic acid sequence comprises at least 11 consecutive nucleotides in SEQ ID NO: 3 or its complementary sequence, and/or at least 11 consecutive nucleotides in SEQ ID NO: 4 or its complementary sequence.

2. The nucleic acid molecule according to claim 1, wherein the nucleic acid sequence comprises SEQ ID NO: 1 or its complementary sequence, and/or SEQ ID NO: 2 or its complementary sequence.

3. The nucleic acid molecule according to claim 2, wherein the nucleic acid sequence comprises SEQ ID NO: 3 or its complementary sequence, and/or SEQ ID NO: 4 or its complementary sequence.

4. The nucleic acid molecule according to claim 3, wherein the nucleic acid sequence comprises SEQ ID NO: 5 or its complementary sequence.

5. A method for detecting the presence of the DNA of a transgenic soybean event DBN9004 in a sample, wherein the method comprises:
contacting a sample to be detected with at least two types of primers for amplifying a target amplification product in a nucleic acid amplification reaction;
performing a nucleic acid amplification reaction; and
detecting the presence of the target amplification product;
the target amplification product comprising at least 11 consecutive nucleotides in SEQ ID NO: 3 or its complementary sequence, and/or at least 11 consecutive nucleotides in SEQ ID NO: 4 or its complementary sequence.

6. The method for detecting the presence of the DNA of a transgenic soybean event DBN9004 in a sample according to claim 5, wherein the target amplification product comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

7. The method for detecting the presence of the DNA of a transgenic soybean event DBN9004 in a sample according to claim 5 or 6, wherein the target amplification product comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence.

8. The method for detecting the presence of the DNA of a transgenic soybean event DBN9004 in a sample according to any one of claims 5-7, wherein at least one of the primers comprises the nucleic acid sequence according to any one of claims 1-4 or a fragment thereof or a complementary sequence thereof.

9. The method for detecting the presence of the DNA of a transgenic soybean event DBN9004 in a sample according to claim 8, wherein the primers comprise a first primer selected from SEQ ID NO: 8 and SEQ ID NO: 10, and a second primer selected from SEQ ID NO: 9 and SEQ ID NO: 11.

10. A method for detecting the presence of the DNA of a transgenic soybean event DBN9004 in a sample, wherein the method comprises:
contacting a sample to be detected with a probe, the probe comprising at least 11 consecutive nucleotides in SEQ ID NO: 3 or its complementary sequence, and/or at least 11 consecutive nucleotides in SEQ ID NO: 4 or its complementary sequence;
hybridizing the sample to be detected with the probe under stringent hybridization conditions; and
detecting the hybridization status between the sample to be detected and the probe.

11. The method for detecting the presence of the DNA of a transgenic soybean event DBN9004 in a sample according to claim 10, wherein the probe comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

12. The method for detecting the presence of the DNA of a transgenic soybean event DBN9004 in a sample according to claim 10 or 11, wherein the probe comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence.

13. The method for detecting the presence of the DNA of a transgenic soybean event DBN9004 in a sample according to any one of claims 10-12, wherein at least one of the probes is labelled with at least one fluorophore.

14. A method for detecting the presence of the DNA of a transgenic soybean event DBN9004 in a sample, wherein the method comprises:
contacting the sample to be detected with a marker nucleic acid molecule, the marker nucleic acid molecule comprising at least 11 consecutive nucleotides in SEQ ID NO: 3 or its complementary sequence, and/or at least 11 consecutive nucleotides in SEQ ID NO: 4 or its complementary sequence;
hybridizing the sample to be detected with the marker nucleic acid molecule under stringent hybridization conditions; and
detecting the hybridization status of the sample to be detected with the marker nucleic acid molecule, and further determining the gene linkage between the glyphosate tolerance and/or glufosinate tolerance and the marker nucleic acid molecule in genetics by the marker-assisted breeding analysis.

15. The method for detecting the presence of the DNA of a transgenic soybean event DBN9004 in a sample according to claim 14, wherein the marker nucleic acid molecule comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

16. The method for detecting the presence of the DNA of a transgenic soybean event DBN9004 in a sample according to claim 14 or 15, wherein the marker nucleic acid molecule comprises at least one selected from: SEQ ID NO: 1 or its complementary sequence, SEQ ID NO: 2 or its complementary sequence, SEQ ID NO: 6 or its complementary sequence, and SEQ ID NO: 7 or its complementary sequence.

17. A DNA detection kit, wherein it comprises at least one DNA molecule, wherein the DNA molecule comprises at least 11 consecutive nucleotides of a homologous sequence of SEQ ID NO: 3 or its complementary sequence, and/or at least 11 consecutive nucleotides of a homologous sequence of SEQ ID NO: 4 or its complementary sequence, and can be served as a DNA primer or a probe specific for the transgenic soybean event DBN9004 or its progenies.

18. The DNA detection kit according to claim 17, wherein the DNA molecule comprises consecutive nucleotides at positions 1-11 or positions 12-22 in SEQ ID NO: 1 or its complementary sequence, and/or consecutive nucleotides at positions 1-11 or positions 12-22 in SEQ ID NO: 2 or its complementary sequence.

19. The DNA detection kit according to claim 17 or 18, wherein the DNA molecule comprises at least one selected from: a homologous sequence of SEQ ID NO: 1 or its complementary sequence, a homologous sequence of SEQ ID NO: 2 or its complementary sequence, a homologous sequence of SEQ ID NO: 6 or its complementary sequence, and a homologous sequence of SEQ ID NO: 7 or its complementary sequence.

20. A plant cell or part, wherein it comprises a nucleic acid sequence encoding a glyphosate-tolerant EPSPS protein, a nucleic acid sequence encoding a glufosinate-tolerant PAT protein, and a nucleic acid sequence of a specific region, the nucleic acid sequence of the specific region comprising at least one selected from the sequences as shown in SEQ ID NO: 1, SEQ ID NO: 2, SEQ ID NO: 6 and SEQ ID NO: 7.

21. A method for producing a soybean plant tolerant to a glyphosate herbicide and/or a glufosinate herbicide, wherein the method comprises introducing into the genome of the soybean plant a nucleic acid sequence encoding a glyphosate-tolerant EPSPS protein and/or a nucleic acid sequence encoding a glufosinate-tolerant PAT protein, and a nucleic acid sequence of a specific region selected from at least one nucleic acid sequence of the sequences as shown in SEQ ID NO: 1, SEQ ID NO: 2, SEQ ID NO: 3, SEQ ID NO: 4, SEQ ID NO: 6 and SEQ ID NO: 7;
preferably, the method comprises introducing into the genome of the soybean plant a nucleic acid sequence as shown in SEQ ID NO: 5.

22. The method for producing a soybean plant tolerant to a glyphosate herbicide and/or a glufosinate herbicide according to claim 21, wherein the method comprises:
sexually hybridizing a first parent soybean plant of the transgenic soybean event DBN9004 which is tolerant to a glyphosate herbicide and/or a glufosinate herbicide with a second parent soybean plant which lacks glyphosate and/or glufosinate tolerance to produce a large number of progeny plants;
treating the progeny plants with a glyphosate herbicide and/or a glufosinate herbicide; and
selecting the progeny plants which are tolerant to glyphosate and/or glufosinate.

23. A method for culturing a soybean plant tolerant to a glyphosate herbicide and/or a glufosinate herbicide, wherein the method comprises:
planting at least one soybean seed, the genome of the soybean seed comprising a nucleic acid sequence encoding a glyphosate-tolerant EPSPS protein and/or a nucleic acid sequence encoding a glufosinate-tolerant PAT protein, and a nucleic acid sequence of a specific region;
growing the soybean seed into a soybean plant; and
spraying the soybean plant with an effective dose of a glyphosate herbicide and/or a glufosinate herbicide and harvesting the plant with reduced plant damage compared to other plants without the nucleic acid sequence of the specific region;
the nucleic acid sequence of the specific region being selected from at least one nucleic acid sequence of the sequences as shown in SEQ ID NO: 1, SEQ ID NO: 2, SEQ ID NO: 3, SEQ ID NO: 4, SEQ ID NO: 6 and SEQ ID NO: 7;
preferably, the method comprises:
planting at least one soybean seed, the genome of the soybean seed comprising a nucleic acid sequence as shown in SEQ ID NO: 5;
growing the soybean seed into a soybean plant; and
spraying the soybean plant with an effective amount of a glyphosate herbicide, and harvesting a plant with reduced plant damage compared to other plants without SEQ ID NO: 5.

24. A method for protecting a plant from damage caused by a herbicide and/or controlling weeds in a field, wherein the method comprises applying a herbicide containing an effective dose of glyphosate and/or glufosinate to a field for planting at least one transgenic soybean plant, wherein the transgenic soybean plant comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome, and the transgenic soybean plant has tolerance to the glyphosate herbicide and/or the glufosinate herbicide.

25. A method for controlling glyphosate resistant weeds in a field for a glyphosate tolerant plant, wherein the method comprises applying a herbicide containing an effective dose of glufosinate to a field for planting at least one glyphosate tolerant transgenic soybean plant, wherein the glyphosate tolerant transgenic soybean plant comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome, and the glyphosate tolerant transgenic soybean plant has tolerance to the glufosinate herbicide at the same time.

26. A method for delaying insect resistance, wherein the method comprises growing at least one transgenic soybean plant having glyphosate and/or glufosinate tolerance in a field for planting an insect resistant soybean plant, wherein the transgenic soybean plant having glyphosate and/or glufosinate tolerance comprises at least one nucleic acid sequence selected from the sequences as shown in SEQ ID NO: 1, SEQ ID NO: 2, SEQ ID NO: 3, SEQ ID NO: 4, SEQ ID NO: 5, SEQ ID NO: 6 and SEQ ID NO: 7 in its genome.

27. An agricultural product or commodity comprising a polynucleotide of SEQ ID NO: 1 or SEQ ID NO: 2, wherein the agricultural product or commodity is lecithin, a fatty acid, glycerol, a sterol, a soybean flake, soy flour, a soy protein or its concentrate, a soybean oil, a soy protein fiber, a soy milk clot or bean curd.


SEQUENCE LISTING

<110>  BEIJING DABEINONG TECHNOLOGY GROUP CO., LTD.
       BEIJING DABEINONG BIOTECHNOLOGY CO., LTD.

<120>  Nucleic Acid Sequence for Detecting Existence of Transgenic Soybean
Event DBN9004 in Biological Sample, Kit Containging Same and Detection
Method Therefor

<130>  FP1170131P

<160>  31

<170>  PatentIn version 3.5

<210>  1
<211>  22
<212>  DNA
<213>  Artificial Sequence

<220>
<223> A sequence with a length of 22 nucleotides located at the 5¡¯ terminus
of the inserted sequence near the insertion conjugation site in the
transgenic soybean event DBN9004

<400>  1
tcacagagtt gtaaacaaat tg                                              22


<210>  2
<211>  22
<212>  DNA
<213>  Artificial Sequence

<220>
<223> A sequence with a length of 22 nucleotides located at the 3¡¯ terminus
of the inserted sequence near the insertion conjugation site in the
transgenic soybean event DBN9004

<400>  2
tttgaagaca tatttttgtg ga                                              22


<210>  3
<211>  1207
<212>  DNA
<213>  Artificial Sequence

<220>
<223> A sequence with a length of 1207 nucleotides located at the 5¡¯
terminus of the inserted sequence near the insertion conjugation site in the
transgenic soybean event DBN9004

<400>  3
cgatagatta cgaataatag cgaaagatct gaaagaaatt tttcatgttc ttttatgaat      60

atgagtttga ctcttcttaa agttatataa aatttcgttg gttggacatt tccttttatg     120

gattaattac gtaattgtga cgacatgaaa ctgcagttta aactcactat aatttcttca     180

ataaatcatt actacacctg acatagtatt tactattttt aaaagtgttt aattttggat     240

tattattagt gtaacgaatt gaattaatta ttttcaaaaa catcactttt ttataaataa     300

aatcctaagg aataatttat tttatgggta gataaaattg attttaagta attgaatgaa 360

atactcatag aaaacattga ataaaattgg aaactatgtc aaaattaatc tcaattatta 420

attcaaatgg gaatacatgc tttttattta ttaaaattac gagaattaaa tttctaacaa 480

aaataaacag gatttgaatt tgtttatcaa acatacaatt acatttttga gtttgatctt 540

gtttatttta aagttgagta aaactatttt tgatataatt aattttaaac tcttggacct 600

gattaaaata tatatatata ttaaaatatg tattcgttat taaaaataca atacaaagaa 660

aatagacggg acttgatgaa agaagagcgt tgcatttacg ctccataaac gtgtgctttc 720

attacggggt cacagagttg taaacaaatt gacgcttaga caacttaata acacattgcg 780

gatacggcca tgctggccgc ccatcatggg cgcgccactg gattttggtt ttaggaatta 840

gaaattttat tgatagaagt attttacaaa tacaaataca tactaagggt ttcttatatg 900

ctcaacacat gagcgaaacc ctataagaac cctaattccc ttatctggga actactcaca 960

cattattata gagagagata gatttgtaga gagagactgg tgatttcagc gggcatgcct 1020

gcaggtcgac tcagatctgg gtaactggcc taactggcct tggaggagct ggcaactcaa 1080

aatccctttg ccaaaaacca acatcatgcc atccaccatg cttgtatcca gctgcgcgca 1140

atgtaccccg ggctgtgtat cccaaagcct catgcaacct aacagatgga tcgtttggaa 1200

ggcctat 1207

<210> 4
<211> 631
<212> DNA
<213> Artificial Sequence

<220>
<223> A sequence with a length of 631 nucleotides located at the 3¡¯
terminus of the inserted sequence near the insertion conjugation site in the
transgenic soybean event DBN9004

<400> 4
gaagaagctg cgctaaaacc ctagccgcaa gagaagaaga gtgaacaaag gtgtgaagag 60

aagtaatgcg acgagggtta gagggtttat ttatagctac aactcttttc ttctgtttac 120

gaaattaccc ttactaattt accctagttc cgtttgtatc cttctctgct ttcgtagcga 180

caattgaaac gctcgctaaa gcgcgtgctt ctcacgtctc gtagttaagc ttgataacgc 240

ggccgctcta gggcgcgccg ggtcccgttt aaactatcag tgtttgaaga catatttttg 300

tggatctagg ttcatcacct acatcttttt ctttctattt taatttaaat atttgtttaa 360

ttctcattaa ttttctttcg gtcatttctt tccacataat tacccattaa atttgagtaa 420

ctacagctct aaaaatcttt tttttaaaat gataattatt acaacaacaa caacaacaac 480

aacgccttat cccactaggt ggggtcgact acatggatca acttccgtca taatgttcta 540

tcaagtacca tacttctatc caaatcatta agttcgaggt ccttcttgat aacctctctt 600

ataatctttt tgggtcttcc tctgcctcga a 631

<210> 5
<211> 7059
<212> DNA
<213> Artificial Sequence

<220>
<223> Entire T-DNA sequence, 5¡¯ and 3¡¯ flanking soybean genome sequences

<400> 5
atgtttccct ttttcccgac ccacttctcc ttctatccct tcctatttat aacacctaat 60

tatctatttt attaaagttt tataaataat tcatttattt aataataata attttttaaa 120

atataattga tagtatttta ttttattttt taattattta tcaatatatt ctttaaattt 180

agagtttttt ttttctaaaa ctgattaata caaaaaacat tataaattaa ttcttctaaa 240

aaattaaata ttattttaaa cttagtattc ttataaattg gaatagagta ttatactact 300

cactgttctg tacctttcaa ctttcaattt ggctccctcc actgcttaaa ttctactctc 360

gtcttccatt actggcgtcc ttaattatgc ctttttctta atttcttcat atctcaactc 420

attcataggc ttttgcttat ttattattac gcaaactcgc ctaacatgta ttttctatta 480

aattaatcga tcttgtttta ctaggacaaa aacatatttg ttagatgtcg atagattacg 540

aataatagcg aaagatctga aagaaatttt tcatgttctt ttatgaatat gagtttgact 600

cttcttaaag ttatataaaa tttcgttggt tggacatttc cttttatgga ttaattacgt 660

aattgtgacg acatgaaact gcagtttaaa ctcactataa tttcttcaat aaatcattac 720

tacacctgac atagtattta ctatttttaa aagtgtttaa ttttggatta ttattagtgt 780

aacgaattga attaattatt ttcaaaaaca tcactttttt ataaataaaa tcctaaggaa 840

taatttattt tatgggtaga taaaattgat tttaagtaat tgaatgaaat actcatagaa 900

aacattgaat aaaattggaa actatgtcaa aattaatctc aattattaat tcaaatggga 960

atacatgctt tttatttatt aaaattacga gaattaaatt tctaacaaaa ataaacagga 1020

tttgaatttg tttatcaaac atacaattac atttttgagt ttgatcttgt ttattttaaa 1080

gttgagtaaa actatttttg atataattaa ttttaaactc ttggacctga ttaaaatata 1140

tatatatatt aaaatatgta ttcgttatta aaaatacaat acaaagaaaa tagacgggac 1200

ttgatgaaag aagagcgttg catttacgct ccataaacgt gtgctttcat tacggggtca 1260

cagagttgta aacaaattga cgcttagaca acttaataac acattgcgga tacggccatg 1320

ctggccgccc atcatgggcg cgccactgga ttttggtttt aggaattaga aattttattg 1380

atagaagtat tttacaaata caaatacata ctaagggttt cttatatgct caacacatga 1440

gcgaaaccct ataagaaccc taattccctt atctgggaac tactcacaca ttattataga 1500

gagagataga tttgtagaga gagactggtg atttcagcgg gcatgcctgc aggtcgactc 1560
agatctgggt aactggccta actggccttg gaggagctgg caactcaaaa tccctttgcc 1620
aaaaaccaac atcatgccat ccaccatgct tgtatccagc tgcgcgcaat gtaccccggg 1680
ctgtgtatcc caaagcctca tgcaacctaa cagatggatc gtttggaagg cctataacag 1740
caaccacaga cttaaaacct tgcgcctcca tagacttaag caaatgtgtg tacaatgtgg 1800
atcctaggcc caacctttga tgcctatgtg acacgtaaac agtactctca actgtccaat 1860
cgtaagcgtt cctagccttc cagggcccag cgtaagcaat accagccaca acaccctcaa 1920
cctcagcaac caaccaaggg tatctatctt gcaacctctc tagatcatca atccactctt 1980
gtggtgtttg tggctctgtc ctaaagttca ctgtagacgt ctcaatgtaa tggttaacga 2040
tatcacaaac cgcggccata tcagctgctg tagctggcct aatctcaact ggtctcctct 2100
ccggagacat ggtggatccc cgggtaccct gtcctctcca aatgaaatga acttccttat 2160
atagaggaag ggtcttgcga aggatagtgg gattgtgcgt catcccttac gtcagtggag 2220
atatcacatc aatccacttg ctttgaagac gtggttggaa cgtcttcttt ttccacgatg 2280
ctcctcgtgg gtgggggtcc atctttggga ccactgtcgg cagaggcatc ttcaacgatg 2340
gcctttcctt tatcgcaatg atggcatttg taggagccac cttccttttc cactatcttc 2400
acaataaagt gacagatagc tgggcaatgg aatccgagga ggtttccgga tattaccctt 2460
tgttgaaaag tctcaattgc cctttggtct tctgagactg tatctttgat atttttggag 2520
tagacaagcg tgtcgtgctc caccatgttg acgaagattt tcttcttgtc attgagtcgt 2580
aagagactct gtatgaactg ttcgccagtc tttacggcga gttctgttag gtcctctatt 2640
tgaatctttg actccatggc cactgcaggt cacgatcgat gcggccgctt cgagtggctg 2700
caggtcgatt gatgcatgtt gtcaatcaat tggcaagtca taaaatgcat taaaaaatat 2760
tttcatactc aactacaaat ccatgagtat aactataatt ataaagcaat gattagaatc 2820
tgacaaggat tctggaaaat tacataaagg aaagttcata aatgtctaaa acacaagagg 2880
acatacttgt attcagtaac atttgcagct tttctaggtc tgaaaatata tttgttgcct 2940
agtgaataag cataatggta caactacaag tgttttactc ctcatattaa cttcggtcat 3000
tagaggccac gatttgacac atttttactc aaaacaaaat gtttgcatat ctcttataat 3060
ttcaaattca acacacaaca aataagagaa aaaacaaata atattaattt gagaatgaac 3120
aaaaggacca tatcattcat taactcttct ccatccattt ccatttcaca gttcgatagc 3180
gaaaaccgaa taaaaaacac agtaaattac aagcacaaca aatggtacaa gaaaaacagt 3240
tttcccaatg ccataatact caaactcagt aggattctgg tgtgtgcgca atgaaactga 3300
tgcattgaac ttgacgaacg ttgtcgaaac cgatgatacg aacgaaagct ctagctagag 3360

gatccggtac cgagctcgaa ttcttgagct catcaagcag ccttagtgtc ggagagttcg 3420
atcttagctc caagaccagc catcaaatcc atgaactctg ggaagctagt agcgatcata 3480
gtagcatcat caacagtaac agggttttca gaaacgagac ccataacgag gaagctcata 3540
gcgatacggt gatcgaggtg ggtagcgaca gctgctccag aagcgttacc gagaccctta 3600
ccgtcaggac gaccacgcac gacgagagaa gtctcacctt catcgcaatc aacaccgttg 3660
agcttgagac cgtttgcgac agcagaaaga cggtcgcttt ccttaacacg gagttcttcc 3720
aaaccgttca taacggtagc accttcagcg aatgcagctg caacagcgag aattggatac 3780
tcgtcgatca tagaaggagc acggtcttct ggaacagtaa cacccttcaa agtagaagaa 3840
cgaacacgca agtcagccac gtcttctcca ccagcaagac gtgggttgat cacttcgatg 3900
tcggcaccca tttcctgcag agtcaagatg agaccagtac gggttgggtt catcaaaacg 3960
ttaaggatgg tgacgtcgga acctggaaca agcaaggcag caaccaatgg gaaagcagta 4020
gaggatggat cacctggaac atcaatcact tgaccggtga gcttaccacg accttcaaga 4080
cggatggtac gcacaccgtc agcatcagtc tcaacggtaa ggttagcacc aaaaccttga 4140
agcatctttt cagtgtggtc acgagtcatg attggctcga taacagtggt gatacctggg 4200
gtgttgagac cagcaagcag aacagcggac ttcacttgag cggaagccat aggtaccctg 4260
taggtgattg gcgttggagt ctttggtcca cgcaaggtaa ctggaagacg atcaccgtct 4320
tcagacttca cctgcacacc catttcgcga agtgggttca acacacgacc cattggacgc 4380
ttagtgagag aagcgtcacc aatgaaagtg ctatcgaaat cgtaaacacc aacaagaccc 4440
atagtcaaac ggcaaccagt tgcagcgtta ccgaaatcga gaggagcctc aggagcaagg 4500
agtccaccgt taccaacacc atcaatgatc caagtatcac cttccttacg gattctggca 4560
cccatagctt gcatagcctt accagtgttg ataacatctt caccttccaa aagaccggtg 4620
atacgagttt caccgctagc gagacctcca aacatgaagg acctgtggga gatagacttg 4680
tcacctggaa tacggacggt tccagaaaga ccagaggact tacgagcagt tgctggacgg 4740
ctgcttgcac cgtgaagcat gcacgccgtg gaaacagaag acatgacctt aagaggacga 4800
agctcagagc caattaacgt catcccactc ttcttcaatc cccacgacga cgaaatcgga 4860
taagctcgtg gatgctgctg cgtcttcaga gaaaccgata agggagattt gcgttgactg 4920
gatttcgaga gattggagat aagagatggg ttctgcacac cattgcagat tctgctaact 4980
tgcgccatgg cttccttaaa tctgcaaaaa tccagaaaaa aaaaaagttg tcaaaaccat 5040
aacacatcaa cacaccatat aaagtaaaaa taaaagttta acaacattaa gacctttgtc 5100
attacaaaac ctttcataag cataaaaaga ttaagatcta aatacccacc atacaacaag 5160
cccaaaaata ggtatgctca atcgtaacgc atcaaactca gtatgattcc aacagataaa 5220
aaaatagaca taacaaaatt tatggcactc ccatacataa gatcgagata aaacaagggc 5280

atgtttagca cactgtgacg tatatagatc tattcatcag taaccaaaaa acttgagctt 5340

gaaccgatta aaagtaacag ataatagatc tattcatcag taaccaaaaa actttagctt 5400

gaatcgatta aagtaacagt taatagatct attcatcagt aaccaaaaac tttcttgaac 5460

caaaaactta tataagatct ggatccaaag atttgaatat aacaagcaaa taaaagcctt 5520

accaaaaaaa ctttaaatca aaagtaataa aaaaaacact tttgaaaaca ccatagcacc 5580

gttattaaca actcgaacga aaagaagaag aataagcaca tactacacaa acaaaattga 5640

tctctgaaac atggataaaa ttgaagacaa cgacgacaga ttcagaaaaa acatattgca 5700

agcaacataa accaatagaa taaacacgaa tagaaggaat aaaatccata gaacggtggt 5760

agataacgaa cctagaagaa gctgcgctaa aaccctagcc gcaagagaag aagagtgaac 5820

aaaggtgtga agagaagtaa tgcgacgagg gttagagggt ttatttatag ctacaactct 5880

tttcttctgt ttacgaaatt acccttacta atttacccta gttccgtttg tatccttctc 5940

tgctttcgta gcgacaattg aaacgctcgc taaagcgcgt gcttctcacg tctcgtagtt 6000

aagcttgata acgcggccgc tctagggcgc gccgggtccc gtttaaacta tcagtgtttg 6060

aagacatatt tttgtggatc taggttcatc acctacatct ttttctttct attttaattt 6120

aaatatttgt ttaattctca ttaattttct ttcggtcatt tctttccaca taattaccca 6180

ttaaatttga gtaactacag ctctaaaaat ctttttttta aaatgataat tattacaaca 6240

acaacaacaa caacaacgcc ttatcccact aggtggggtc gactacatgg atcaacttcc 6300

gtcataatgt tctatcaagt accatacttc tatccaaatc attaagttcg aggtccttct 6360

tgataacctc tcttataatc tttttgggtc ttcctctgcc tcgaattgtt tgctttctct 6420

ccatctggtc tactctcctc actacagagt ctaccggttt tctctctaca tgcccaaacc 6480

acctaagtct attttccacc atcttatcta caataggcgc tactccaacc ctctctctaa 6540

tagctccgtt tctaatttta tcctgtcgag tcttaccaca catccaccgc aacatcctca 6600

tctccactac acctacttta gtctcatgtt ggctcttgac cgcccaacat tctgttccgt 6660

acaaaatcgc cagtcttacc gcagtcccat aaaactttcc ctttagcttg atcggtacct 6720

ttgcatcaca taacaccccc gatgcttttc tccatttcat caatcctgct tgaatgcgat 6780

gattcacatc cccttcaatt tctccatcat cctgtattac agacccaaga tatttaaacc 6840

gtgtgacttg agggataata tggtctccta ttttcacctc tgagttagat accctcctac 6900

ttttgttgaa cttacattcc atatactccg atttgcttct gcttaggcga aagccatgtg 6960

tttctagagc tcgtctccaa gtttccaacc tctcattcaa ctcctccctc gactctccaa 7020

ggaggactat gtcatctaca aaaagcatgc atctcggcg 7059

<210> 6

<211>  132
<212>  DNA
<213>  Artificial Sequence

<220>
<223>  A sequence which is located within SEQ ID NO: 3 and spans the
nucleotide sequence in the pDBN4003 construct DNA sequence and t35S
transcription termination sequence

<400>  6
taaacaaatt gacgcttaga caacttaata acacattgcg gatacggcca tgctggccgc      60

ccatcatggg cgcgccactg gattttggtt ttaggaatta gaaattttat tgatagaagt     120

attttacaaa ta                                                         132


<210>  7
<211>  118
<212>  DNA
<213>  Artificial Sequence

<220>
<223>  A sequence which is located within SEQ ID NO: 4 and spans the
prGm17gTsf1 promoter sequence and the nucleotide sequence in the pDBN4003
construct DNA sequence

<400>  7
tttcgtagcg acaattgaaa cgctcgctaa agcgcgtgct tctcacgtct cgtagttaag      60

cttgataacg cggccgctct agggcgcgcc gggtcccgtt taaactatca gtgtttga       118


<210>  8
<211>  20
<212>  DNA
<213>  Artificial Sequence

<220>
<223>  A first primer amplifying SEQ ID NO: 3

<400>  8
cgatagatta cgaataatag                                                  20


<210>  9
<211>  20
<212>  DNA
<213>  Artificial Sequence

<220>
<223>  A second primer amplifying SEQ ID NO: 3

<400>  9
ataggccttc caaacgatcc                                                  20


<210>  10
<211>  20
<212>  DNA
<213>  Artificial Sequence

<220>

<223>  A first primer amplifying SEQ ID NO: 4

<400>  10
gaagaagctg cgctaaaacc                                                    20


<210>  11
<211>  20
<212>  DNA
<213>  Artificial Sequence

<220>
<223>  A second primer amplifying SEQ ID NO: 4

<400>  11
ttcgaggcag aggaagaccc                                                    20


<210>  12
<211>  25
<212>  DNA
<213>  Artificial Sequence

<220>
<223>  A primer on 5¡¯ flanking genome sequence

<400>  12
acaatacaaa gaaaatagac gggac                                              25


<210>  13
<211>  25
<212>  DNA
<213>  Artificial Sequence

<220>
<223>  A primer located on T-DNA and paired with SEQ ID NO: 12

<400>  13
gagcatataa gaaaccctta gtatg                                              25


<210>  14
<211>  21
<212>  DNA
<213>  Artificial Sequence

<220>
<223>  A primer on 3¡¯ flanking genome sequence, which is paired with SEQ ID
NO: 12 to detect whether the transgene is a homozygote or a heterozygote

<400>  14
gggcatgtag agagaaaacc g                                                  21


<210>  15
<211>  20
<212>  DNA
<213>  Artificial Sequence

<220>
<223>  A primer located on T-DNA and paired with SEQ ID NO: 14

<400>  15
attgaaacgc tcgctaaagc                                                   20


<210>  16
<211>  23
<212>  DNA
<213>  Artificial Sequence

<220>
<223>  Taqman primer 1 for detecting EPSPS

<400>  16
ttggtgctaa ccttaccgtt gag                                               23


<210>  17
<211>  22
<212>  DNA
<213>  Artificial Sequence

<220>
<223>  Taqman primer 2 for detecting EPSPS

<400>  17
gcttaccacg accttcaaga cg                                                22


<210>  18
<211>  26
<212>  DNA
<213>  Artificial Sequence

<220>
<223>  Taqman probe 1 for detecting EPSPS

<400>  18
ctgatgctga cggtgtgcgt accatc                                            26


<210>  19
<211>  24
<212>  DNA
<213>  Artificial Sequence

<220>
<223>  Taqman primer 3 for detecting PAT

<400>  19
cagttgagat taggccagct acag                                              24


<210>  20
<211>  27
<212>  DNA
<213>  Artificial Sequence

<220>
<223>  Taqman primer 4 for detecting PAT

<400>  20
ttcactgtag acgtctcaat gtaatgg                                           27

<210>  21
<211>  24
<212>  DNA
<213>  Artificial Sequence

<220>
<223>  Taqman probe 2 for detecting PAT

<400>  21
cagctgatat ggccgcggtt tgtg                                              24


<210>  22
<211>  25
<212>  DNA
<213>  Artificial Sequence

<220>
<223>  A first primer of soybean endogenous gene of ubiquitin

<400>  22
agcatttgaa ctacattctc cttga                                             25


<210>  23
<211>  23
<212>  DNA
<213>  Artificial Sequence

<220>
<223>  A second primer of soybean endogenous gene of ubiquitin

<400>  23
tgcagctaca caaatctaaa tcc                                               23


<210>  24
<211>  506
<212>  DNA
<213>  Artificial Sequence

<220>
<223>  A probe of EPSPS in Southern blot hybridization detection

<400>  24
ccacgacctt caagacggat ggtacgcaca ccgtcagcat cagtctcaac ggtaaggtta      60

gcaccaaaac cttgaagcat cttttcagtg tggtcacgag tcatgattgg ctcgataaca     120

gtggtgatac ctggggtgtt gagaccagca agcagaacag cggacttcac ttgagcggaa     180

gccataggta ccctgtaggt gattggcgtt ggagtctttg gtccacgcaa ggtaactgga     240

agacgatcac cgtcttcaga cttcacctgc acacccattt cgcgaagtgg gttcaacaca     300

cgacccattg gacgcttagt gagagaagcg tcaccaatga aagtgctatc gaaatcgtaa     360

acaccaacaa gacccatagt caaacggcaa ccagttgcag cgttaccgaa atcgagagga     420

gcctcaggag caaggagtcc accgttacca acaccatcaa tgatccaagt atcaccttcc     480

ttacggattc tggcacccat agcttg                                          506

<210>  25
<211>  310
<212>  DNA
<213>  Artificial Sequence

<220>
<223>  A probe of PAT in Southern blot hybridization detection

<400>  25
cagacttaaa accttgcgcc tccatagact taagcaaatg tgtgtacaat gtggatccta       60

ggcccaacct ttgatgccta tgtgacacgt aaacagtact ctcaactgtc caatcgtaag      120

cgttcctagc cttccagggc ccagcgtaag caataccagc cacaacaccc tcaacctcag      180

caaccaacca agggtatcta tcttgcaacc tctctagatc atcaatccac tcttgtggtg      240

tttgtggctc tgtcctaaag ttcactgtag acgtctcaat gtaatggtta acgatatcac      300

aaaccgcggc                                                             310


<210>  26
<211>  25
<212>  DNA
<213>  Artificial Sequence

<220>
<223>  A primer located on T-DNA in the same direction with SEQ ID NO: 13

<400>  26
gggttcttat agggtttcgc tcatg                                             25


<210>  27
<211>  23
<212>  DNA
<213>  Artificial Sequence

<220>
<223>  A primer located on T-DNA in the opposite direction to SEQ ID NO: 13
and used to obtain a flanking sequence

<400>  27
gcctcatgca acctaacaga tgg                                               23


<210>  28
<211>  21
<212>  DNA
<213>  Artificial Sequence

<220>
<223>  A primer located on T-DNA in the opposite direction to SEQ ID NO: 13
and used to obtain a flanking sequence

<400>  28
ccaacatcat gccatccacc a                                                 21


<210>  29

<211>  27
<212>  DNA
<213>  Artificial Sequence

<220>
<223>  A primer located on T-DNA in the same direction with SEQ ID NO: 15

<400>  29
cctagttccg tttgtatcct tctctgc                                          27


<210>  30
<211>  29
<212>  DNA
<213>  Artificial Sequence

<220>
<223>  A primer located on T-DNA in the opposite direction to SEQ ID NO: 15
and used to obtain a flanking sequence

<400>  30
gtttgtgtag tatgtgctta ttcttcttc                                        29


<210>  31
<211>  23
<212>  DNA
<213>  Artificial Sequence

<220>
<223>  A primer located on T-DNA in the opposite direction to SEQ ID NO: 15
and used to obtain a flanking sequence

<400>  31
ctgaatctgt cgtcgttgtc ttc                                              23